(19) Europäisches Patentamt
European Patent Office
Office européen des brevets

(11) Veröffentlichungsnummer: **0 225 608**
**A2**

# (12) EUROPÄISCHE PATENTANMELDUNG

(21) Anmeldenummer: **86116858.1**

(22) Anmeldetag: **04.12.86**

(51) Int. Cl.⁴: **A61K 31/08**, A61K 31/66, C07F 9/09, C07F 9/24

(30) Priorität: **04.12.85 DE 3542893**
**28.02.86 DE 3606631**

(43) Veröffentlichungstag der Anmeldung:
**16.06.87 Patentblatt 87/25**

(84) Benannte Vertragsstaaten:
**ES GR**

(71) Anmelder: **Max-Planck-Gesellschaft zur Förderung der Wissenschaften e.V.**
**Bunsenstrasse 10**
**D-3400 Göttingen(DE)**

(72) Erfinder: **Eibl, Hansjörg, Dr.**
**Steinweg 51**
**D-3406 Bovenden(DE)**

(74) Vertreter: **Huber, Bernhard, Dipl.-Chem. et al**
**Patentanwälte H. Weickmann, Dr. K. Fincke F.A. Weickmann, B. Huber Dr. H. Liska, Dr. J. Prechtel Möhlstrasse 22 Postfach 860 820**
**D-8000 München 86(DE)**

## (54) Arzneimittel.

(57) Cytotoxisch wirksame Arzneimittel, die Verbindungen der Formel I

$$R\text{-}Y\text{-}PO_2^{\ominus}\text{-}X\text{-}R_1 \qquad I$$

oder ein physiologisch verträgliches Salz hiervon enthalten, wobei in der Formel I

R einen gesättigten oder ungesättigten Kohlenwasserstoffrest mit 12 bis 24 C-Atomen bedeutet, der auch halogensubstituiert sein kann,

X ein Sauerstoffatom, NH oder $NR_2$ und Y ein Sauerstoffatom oder NH ist, $R_1$ eine $C_1$-$C_8$-Alkylgruppe ist, oder worin $R_1$ eine $C_2$-$C_8$-Alkylgruppe darstellt, die ungesättigt und/oder mit Halogen, Amino, $C_1$-$C_6$-Alkylamino, Di-$C_1$-$C_6$-alkylamino, Tri-$C_1$-$C_6$-alkylamino, Hydroxy, Carboxy, $C_3$-$C_8$-Cycloalkyl oder Phenyl substituiert ist, und worin $R_1$ außerdem auch 2-tert.-Butyloxycarbonylaminoethyl, 2-tert.-butyloxycarbonylethyl, 2,3-Isopropylidendioxy-propyl-(1), 2,3-Dibenzyloxy-propyl-(1), 1,3-Dibenzyloxy-propyl-(2) oder N-$C_1$-$C_6$-Alkylamino-$C_2$-$C_6$-alkyl bedeuten kann, wenn X ein Sauerstoffatom ist, und worin $R_1$ außerdem auch 2,3-Dihydroxypropyl-(1) bedeuten kann, wenn X die NH-Gruppe ist,

und $R_2$ eine 2,3-Dihydroxypropyl-(1)-gruppe, eine $C_1$-$C_8$-Alkylgruppe oder eine $C_2$-$C_8$-Alkylgruppe, die ungesättigt und/oder mit Halogen, Amino, $C_1$-$C_6$-Alkylamino, Di-$C_1$-$C_6$-alkylamino, Tri-$C_1$-$C_6$-alkylamino, Hydroxy, Carboxy, $C_3$-$C_8$-Cycloalkyl oder Phenyl substituiert ist, darstellt,

Verbindungen der Formel I sowie Verfahren zu deren Herstellung.

EP 0 225 608 A2

# Arzneimittel

Die Europa-Anmeldung 108 565 betrifft Verbindungen der Formel

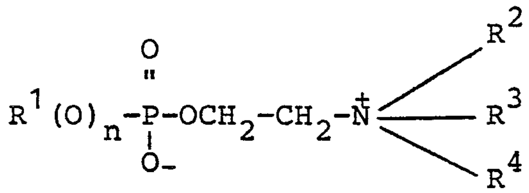

worin $R^1$ ein aliphatischer $C_8$-$C_{30}$-Kohlenwasserstoffrest ist und $R^2$, $R^3$ und $R^4$ Wasserstoff oder niedere Alkylreste sind oder die Gruppe

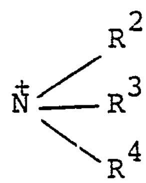

einen cyclischen Ammoniumrest bedeutet, und n = 0 oder 1 ist. Für diese Verbindungen wird angegeben, daß sie die Vermehrung von Tumorzellen hemmen und die Lebenszeit von warmblütigen Tieren, welche Tumore haben, verlängern; außerdem wird eine antifungide Wirkung erwähnt.

Die Erfindung betrifft Arzneimittel, welche sich besonders zur Behandlung von Tumoren eignen.

Die erfindungsgemäßen Arzneimittel gemäß Anspruch 1 besitzen zum Beispiel gegenüber den Mitteln, die aus der Europa-Anmeldung 108 585 bekannt sind, eine verbesserte Wirkung.

Es ist bekannt, daß bisher kein in jeder Hinsicht zufriedenstellendes Arzneimittel zur Behandlung von Tumoren, insbesondere von bösartigen Tumoren, zur Verfügung steht. So ist beispielsweise zur topischen Behandlung von Hautmetastasen bei Patienten mit metastasierenden Tumoren derzeit lediglich 5-Fluorouracil verfügbar. Weiterentwicklungen anderer Cytostatika sind für diese Applikationsart bisher nicht bis zur Klinikreife verfolgt worden. Andererseits ist aus klinischer Sicht ein solches Konzept im palliativen Therapieansatz besonders erwünscht, da alternative Behandlungskonzepte wie chirurgische Maßnahmen, Strahlentherapie und systemische Chemotherapie, vergleichsweise aggressive Therapiemodalitäten darstellen. Außerdem liegt eine beträchtliche Zahl von Patienten als potentielle Behandlungskandidaten für eine solche topische Behandlung vor. So beträgt z. B. der Anteil der Mamma-Karzinom-Patienten, die einen Hautbefall aufweisen, etwa 25 bis 35 %.

Die Voraussetzung zur topischen Behandlung seitens des einzusetzenden Wirkstoffes sind Verträglichkeit auf der Haut, cytotoxische Wirksamkeit gegen Tumorzellen und ausreichende Tiefenpenetration.

Ziel der Erfindung ist daher in erster Linie, ein Arzneimittel zu schaffen, welches zur topischen Behandlung von Tumoren geeignet ist. Ein weiteres Ziel der Erfindung ist es darüberhinaus, ein generell auch in anderen Applikationsformen anwendbares Arzneimittel zu schaffen, welches eine gute Wirksamkeit gegen Tumoren mit geringer Toxizität verbindet und daher allgemein in der Tumortherapie einsetzbar ist.

Diese Aufgaben werden erfindungsgemäß gelöst durch ein Arzneimittel, welches dadurch gekennzeichnet ist, daß es als Wirkstoff wenigstens eine Verbindung der allgemeinen Formel I beziehungsweise I′ gemäß den Ansprüchen 1 und 8

$R\text{-}Y\text{-}PO_2^{\ominus}\text{-}X\text{-}R_1$

oder ein physiologisch verträgliches Salz hiervon, gegebenenfalls zusammen mit üblichen pharmakologischen Zusatz-und Verdünnungsmitteln enthält. Vorzugsweise kommen als Wirkstoffe in Frage: Hexadecylphosphocholin, Oleylphosphocholin, Hexadecylphosphorsäure-(N,N)-bis-(2-chlorethyl)-amid.

Die Formeln I und I′ umfassen auch die möglichen Enantiomere und Diasteromere. Falls die Verbindungen Racemate sind, können diese in an sich bekannter Weise, zum Beispiel mittels einer optisch aktiven Säure, in die optisch aktiven Isomere gespalten werden. Bevorzugt werden jedoch von vornherein enantiomere oder gegebenenfalls diastereomere Ausgangsstoffe eingesetzt, wobei dann als Endprodukt eine entsprechende reine optisch aktive beziehungsweise diastereomere Verbindung erhalten wird.

Im Rahmen der Erfindung ist R vorzugsweise eine Alkylgruppe der angegebenen Kettenlänge, die mit dem Sauerstoff des Glykolrestes über ein endständiges C-Atom oder auch über ein C-Atom innerhalb der Alkylkette verknüpft ist (zum Beispiel über das C-Atom 2 oder C-Atom 3 oder ein anderes mittleres C-Atom). Diese Alkylkette kann geradkettig oder verzweigt sein. Die Alkylkette R kann eine, zwei oder drei Kohlenstoff-Doppelbindungen oder Dreifachbindungen, die auch gemischt vorliegen können, enthalten und/oder Halogensubstituenten aufweisen. Als Halogenatome kommen in Frage: Fluor, Chlor oder Brom. In der Kette R können ein bis drei solcher Halogenatome vorliegen, wobei diese sich an einem oder an verschiedenen C-Atomen des Restes R befinden können.

Bevorzugt werden neben den gesättigten geradkettigen Alkylresten solche mit ein oder zwei Kohlenstoffdoppelbindungen im Molekül. Besonders bevorzugt werden solche Substituenten R, welche einen Alkylrest mit 14 bis 20, vorzugsweise 15 bis 20, insbesondere 16 bis 20 C-Atomen oder einen entsprechenden Alkenylrest mit 14 bis 20, vorzugsweise 15 bis 20, insbesondere 16 bis 20 C-Atomen enthalten.

Beispiele für halogensubstituierte Reste R sind:

Chlorhexadecyl, Bromhexadecyl, Fluorhexadecyl, 9,10-Dibromoctadecyl, 2,3-Dibromoctadecyl, 15,16-Dibromhexadecyl, Bromtetradecyl.

Beispiele für ungesättigte Reste R sind:

9-Octadecenylrest (Oleylalkoholrest, insbesondere bedeutet in der Formel I beziehungsweise I' R diesen 9-Octadecenylrest), 15-Hexadecenylrest, 9,12-Octadecadienylrest (Linoleylrest).

Falls mehr als eine Doppel-oder Dreifachbindung vorliegt, sind diese konjungiert.

Beispiele für gesättigte und unsubstituierte Reste R sind: Tetradecylrest, Hexadecylrest, Octadecylrest.

Falls $R_1$ beziehungsweise $R_2$ eine unsubstituierte Alkylgruppe bedeuten, besteht diese zum Beispiel aus 1 - 6, vorzugsweise 1 -4 C-Atomen. Falls $R_1$ beziehungsweise $R_2$ eine ungesättigte Alkylgruppe bedeutet, besteht sie insbesondere aus 3 bis 6 C-Atomen, wobei zwischen der ungesättigten Funktion einer solchen ungesättigten Alkylgruppe und X mindestens eine einfache C-C-Bindung vorliegen muß. Insbesondere handelt es sich um $C_3$-$C_6$-Alkenylgruppen. Beispiele hierfür sind: Allyl, Butenyl, Pentenyl, Hexenyl.

Falls $R_1$ beziehungsweise $R_2$ substituiert sind, handelt es sich insbesondere um geradkettige Alkyl-oder Alkenylreste, vorzugsweise besteht $R_1$ in diesem Fall aus 2 -6 C-Atomen, wobei die angegebenen Substituenten vorzugsweise in der $\omega$-Stellung der Alkyl-beziehungsweise Alkenylgruppe $R_1$ beziehungsweise $R_2$ stehen; beispielsweise handelt es sich um den Ethyl-oder geraden Propylrest mit einem der angegebenen Substituenten in $\omega$-Stellung (das heißt in 2-Stellung bei Ethyl und 3-Stellung bei Propyl). Falls $R_1$ ein 2-tert.-Butyloxycarbonylaminoethyl-Rest oder ein 2-tert.-Butyloxycarbonylethyl-Rest ist, handelt es sich vorzugsweise um die D-oder L-Form.

Unter den Substituenten von $R_1$ werden die Trialkylammoniumethylreste bevorzugt, wenn X ein Sauerstoffatom ist, wobei die Trialkylreste vorzugsweise jeweils aus einem, zwei oder drei C-Atomen bestehen, vorzugsweise handelt es sich um Methylgruppen. Besonders bevorzugt ist daher der Trimethylammonium-ethylrest. In dieser besonders bevorzugten Ausführungsform handelt es sich bei den Verbindungen der Formel I um Phosphatidylcholinderivate.

Im Falle des $C_3$-$C_8$-Cycloalkyl-Substituenten besteht dieser insbesondere aus 3 -6 C-Atomen (zum Beispiel Cyclopropyl bis Cyclohexyl). Bei der 2,3-Dihydroxy-propyl-(1)-gruppe handelt es sich insbesondere um die sn-1,2-dihydroxypropylamino-(3)-Struktur oder um die sn-2,3-dihydroxypropylamino-(1)-Struktur.

Die Wirkstoffe gemäß dem Stoffanspruch 8 sind neue Verbindungen. Von diesen neuen Verbindungen kommen als Wirkstoffe gemäß der Erfindung vorzugsweise die folgenden in Frage:

Oleyl-phospho-(N,N,N-trimethyl)-propanolamin,
Oleyl-phospho-(N,N,N-trimethyl)-butanolamin,
Oleyl-phospho-(N,N,N-trimethyl)-pentanolamin,
Oleylphosphoserin, Oleyl-phosphoethanolamin,
Oleyl-phosphopropanolamin, Oleyl-phosphobutanolamin,
Oleyl-phosphoglycerin, Hexadecyl-phospho-(N,N,N-trimetyl) -propanolamin.

Als Salze kommen innere Salze in Frage (zum Beispiel wenn $R_1$ eine Trimethylammonio-Alkylgruppe bedeutet) oder Salze mit physiologisch verträglichen Kationen. Die erfindungsgemäßen Arzneimittel beziehungsweise Verbindungen können als innere Salze vorliegen, zum Beispiel wenn $R_1$ eine Aminogruppe enthält. Falls keine inneren Salze vorliegen, beziehungsweise der Rest $R_1$ keine basische Gruppe enthält, wird die negative Ladung der Phosphorsäuregruppe durch ein physiologisch verträgliches Kation abgesättigt. Als solche physiologisch verträglichen Kationen kommen zum Beispiel in Frage: Alkalikationen - (Na, K), Erdalkalikationen (Mg, Ca) oder die Kationen von organischen Aminen, wie zum Beispiel Guanidinium-, Morpholinium, Cyclohexylammoniumkation, Ethylendiammoniumkation, Piperazoniumkation - (in beiden letzteren Fällen ein-oder zweibasisch) oder das Kation, welches sich von einem Amin der Formel $NR_aR_bR_c$ ableitet, worin die Reste $R_a$bis $R_c$ gleich oder verschieden sind und Wasserstoff, $C_1$-$C_2$-Alkylgrup-

pen oder Oxyethylgruppen bedeuten. Falls es sich um Kationen handelt, welche sich von einem Amin der Formel $R_aR_bR_c$ ableiten, handelt es sich vorzugsweise um das Ammoniumkation oder um ein mit ein bis drei $C_1$-$C_2$-Alkylgruppen substituiertes Ammoniumkation oder um ein mit ein bis drei 2-Hydroxyethylgruppen substituiertes Ammoniumkation.

Die Herstellung der Wirkstoffe gemäß der allgemeinen Formel I beziehungsweise I' kann nach an sich bekannten Methoden erfolgen. Das Grundgerüst läßt sich leicht durch Umsetzung einer Verbindung der Formel ROH oder einem funktionellen Derivat davon mit Phosphoroxychlorid und Triethylamin, Umsetzung des Produktes mit einer Verbindung $HXR_1$ und saure Spaltung, wobei R, $R_1$ und X die obige Bedeutung besitzen, erhalten.

Das Herstellungsverfahren für die Verbindungen der Formel I und I' ist in den folgenden Reaktionsgleichungen beispielhaft schematisch erläutert: (Die Gruppe $OCH_3$ in den entsprechenden Formeln steht dabei stellvertretend für die Gruppe OZ.)

1)

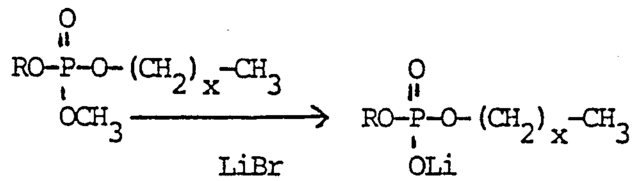


Alkylphosphoalkylester

(x = 1 - 7)

2)

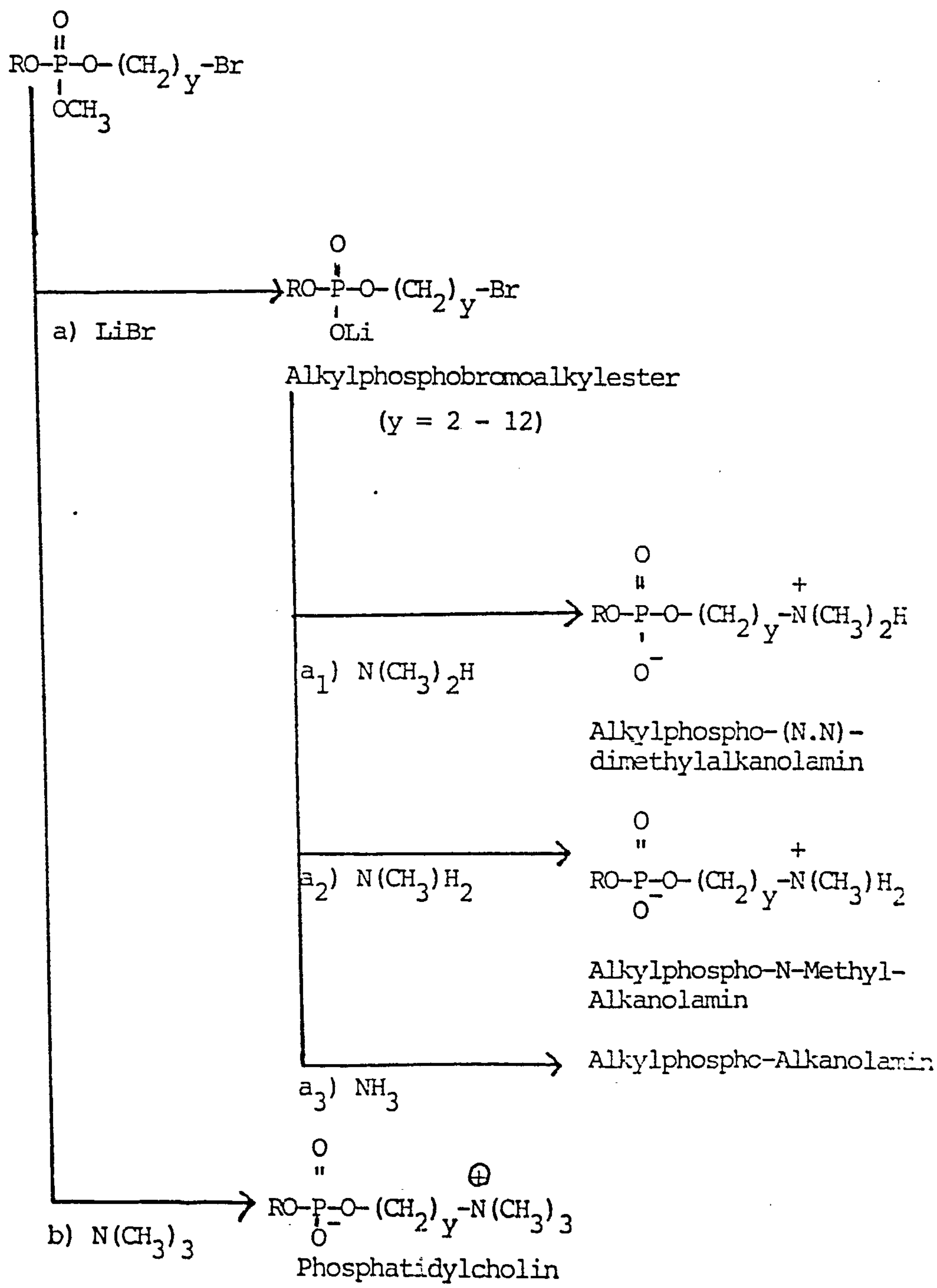


Alkylphosphobromoalkylester

(y = 2 - 12)

Alkylphospho-(N.N)-dimethylalkanolamin

Alkylphospho-N-Methyl-Alkanolamin

Alkylphospho-Alkanolamin

Phosphatidylcholin

3)

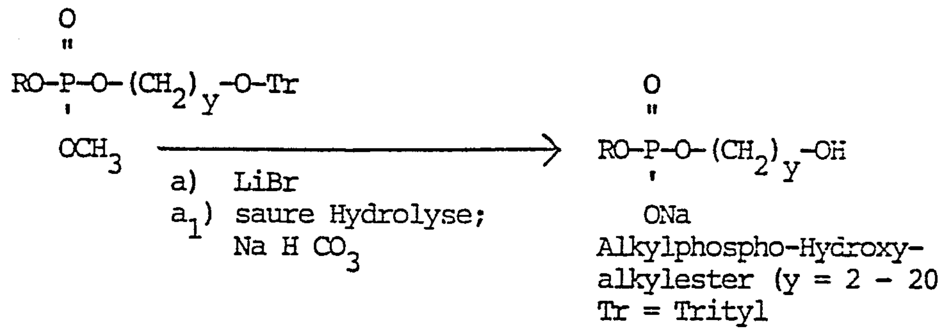


Alkylphospho-Hydroxy-alkylester (y = 2 - 20);
Tr = Trityl

4)

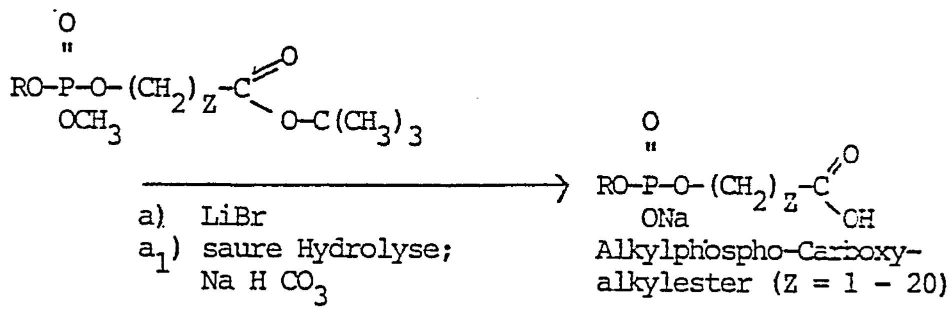


Alkylphospho-Carboxy-alkylester (Z = 1 - 20)

5)

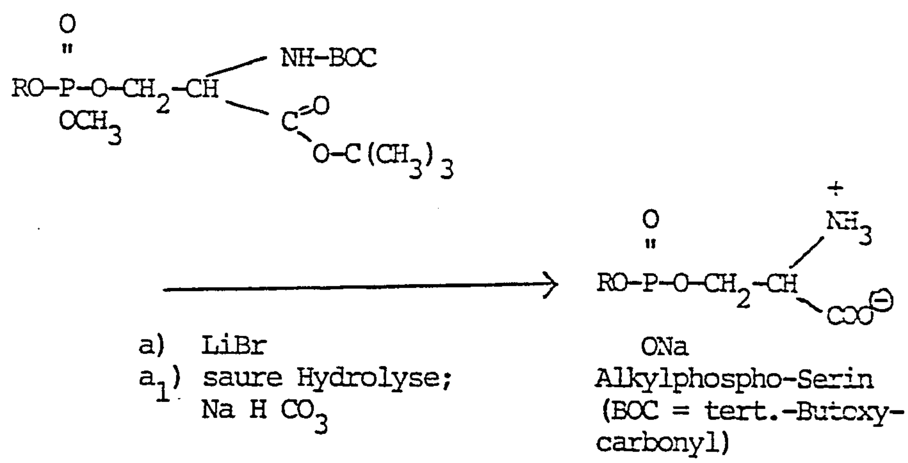


Alkylphospho-Serin
(BOC = tert.-Butoxy-carbonyl)

6) 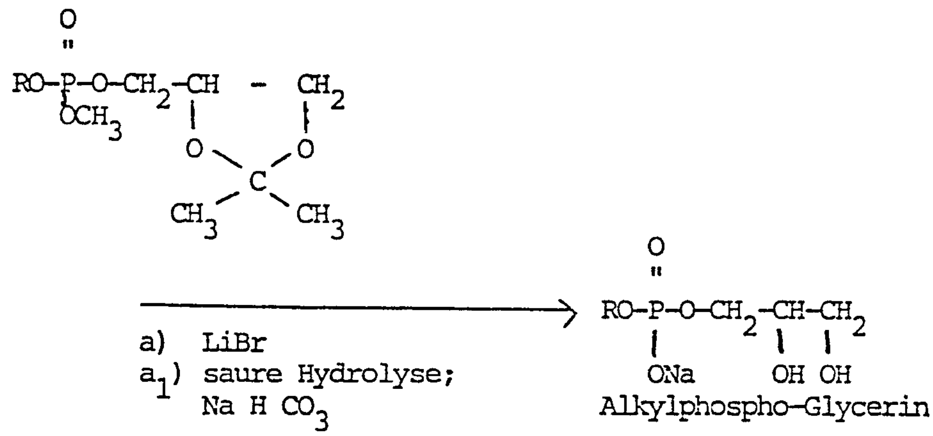


Alkylphospho-Glycerin

7) 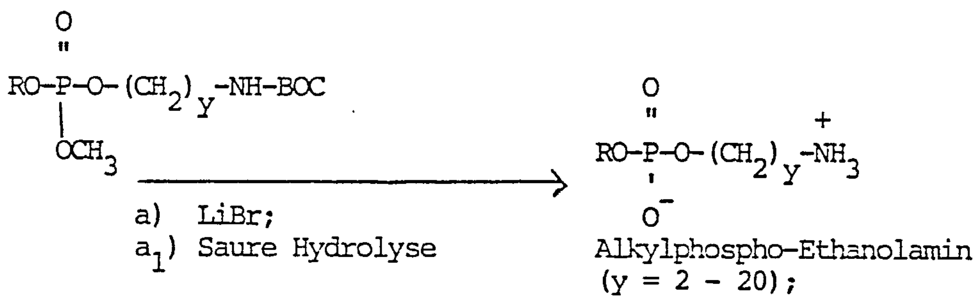


Alkylphospho-Ethanolamin
(y = 2 - 20);
BOC = Butoxycarbonyl

8) 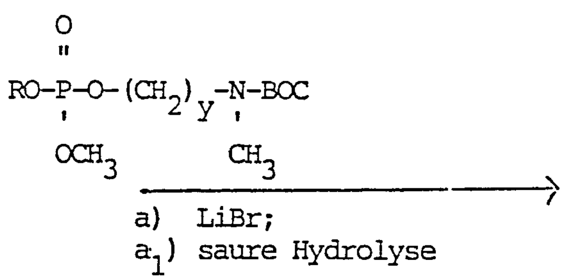


Alkylphospho-N-Methyl-ethanolamin (y = 2 - 20)
BOC = Butoxycarbonyl

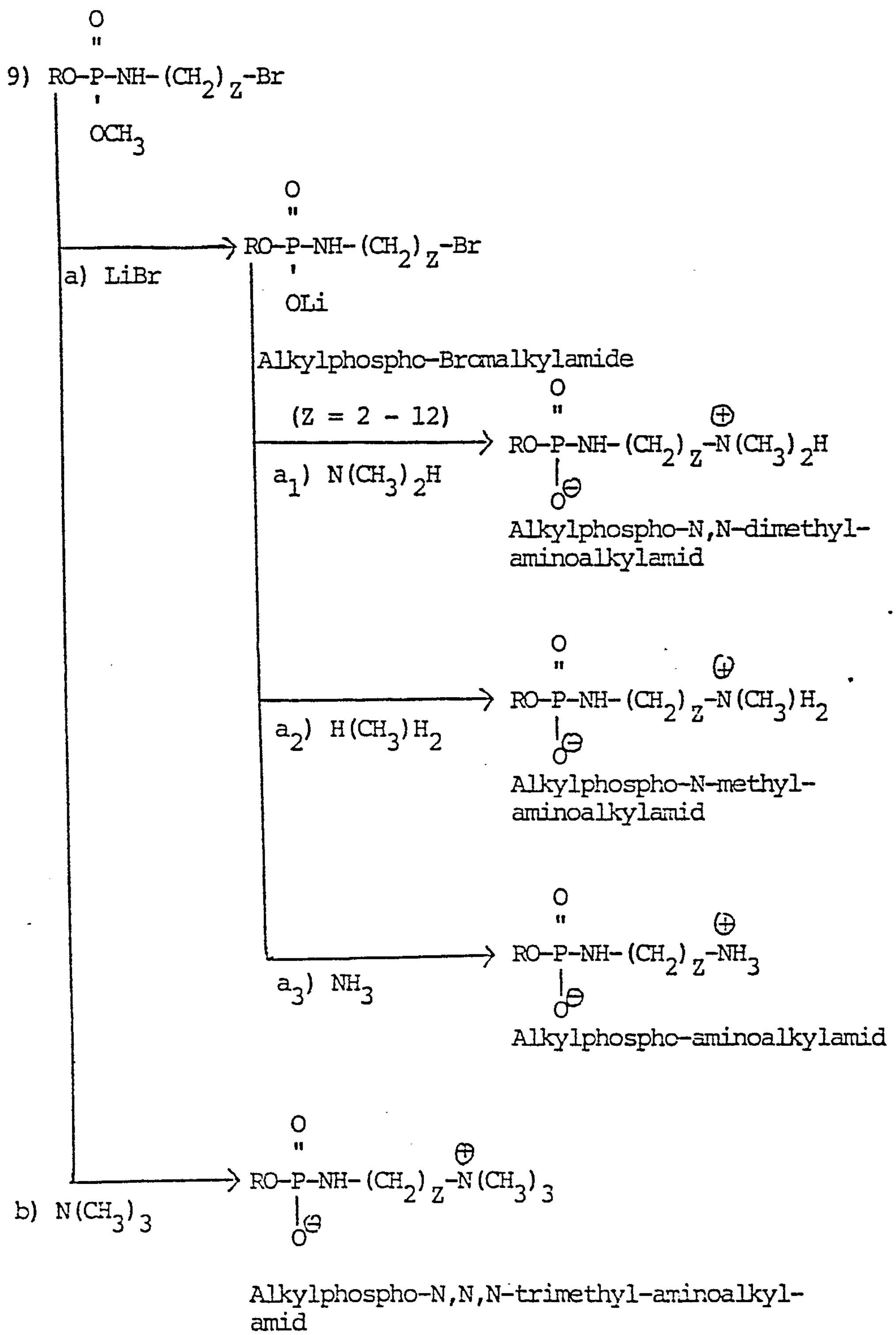
9)
a) LiBr
Alkylphospho-Bromalkylamide
(Z = 2 - 12)
a1) N(CH3)2H
Alkylphospho-N,N-dimethyl-aminoalkylamid
a2) H(CH3)H2
Alkylphospho-N-methyl-aminoalkylamid
a3) NH3
Alkylphospho-aminoalkylamid
b) N(CH3)3
Alkylphospho-N,N,N-trimethyl-aminoalkyl-amid

10) 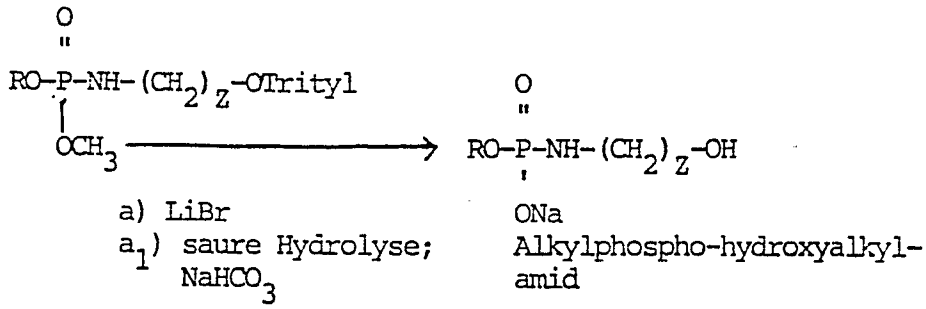


Alkylphospho-hydroxyalkylamid

11) 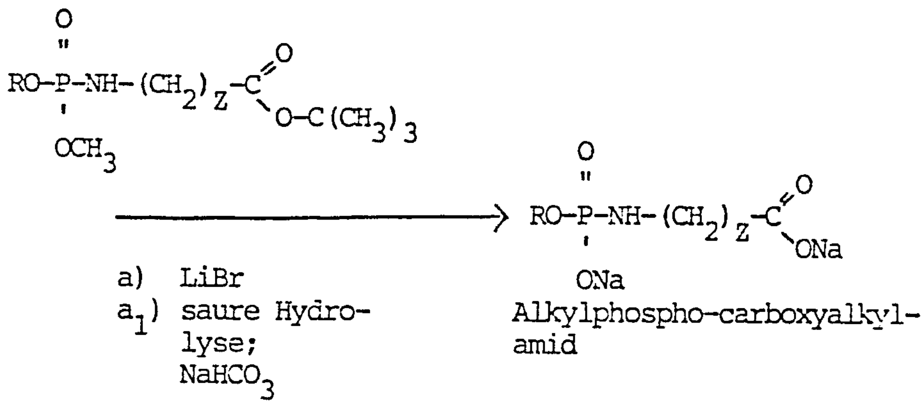


Alkylphospho-carboxyalkylamid

12) 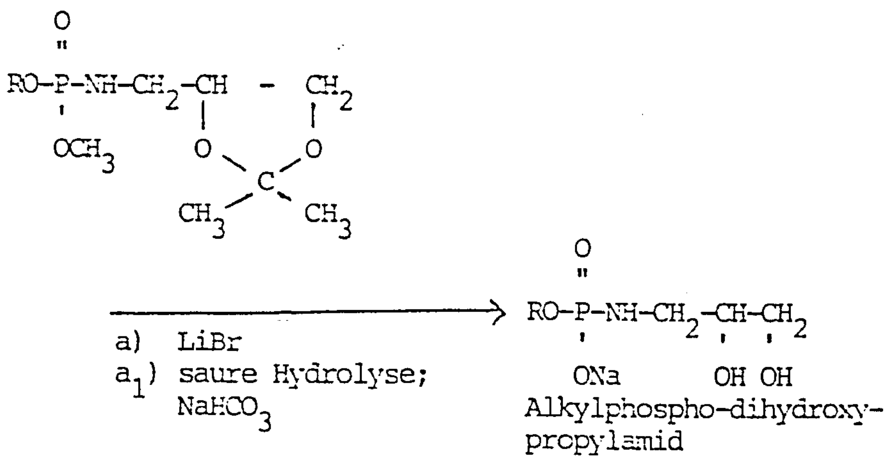


Alkylphospho-dihydroxypropylamid

Weitere Angaben zu dem erfindungsgemäßen Verfahren:

In den Ausgangsstoffen der Formel III können vorhandene Hydroxygruppen, Carboxygruppen, Aminogruppen oder $C_1$-$C_6$-Alkylaminogruppen, die in dem Rest R' oder auch in dem Rest $R^2$ (falls X die Gruppe $NR^2$ ist) vorkommen, durch übliche Schutzgruppen geschützt sein. Benachbarte Hydroxygruppen können durch Ketalisierung mit einem aliphatischen gesättigten $C_3$-$C_6$-Keton geschützt sein.

Es handelt sich hierbei um Reste, die durch Hydrolyse oder Hydrogenolyse leicht abspaltbar sind und während oder nach der Reaktion abgespalten werden. Falls solche Schutzgruppen bei der Verfahrensreaktion nicht abgespalten werden, dann erfolgt eine Abspaltung nach der Reaktion. Häufig enthalten die Ausgangsverbindungen aufgrund ihrer Herstellung bereits derartige Schutzgruppen.

Bei diesen Schutzgruppen handelt es sich beispielsweise um leicht solvolytisch abspaltbare Acylgruppen oder hydrierend abspaltbare Gruppen. Die solvolytisch abspaltbaren Schutzgruppen werden beispielsweise durch Verseifung mit verdünnten Säuren (zum Beispiel Essigsäure, Perchlorsäure, Salzsäure, Schwefelsäure, Ameisensaure, Trifluoressigsäure). oder mittels basischer Substanzen (Pottasche, Soda, wässrige Alkalilösungen, alkoholische Alkalilösungen, $NH_3$) bei Temperaturen zwischen -50 und 150° C, insbesondere zwischen 0 und 100° C, abgespalten. Hydrierend abspaltbare Gruppen wie Arylalkylreste -(Benzylrest) oder Hydroxycarbonylreste (Carbobenzoxyrest) werden zweckmäßig durch katalytische Hydrierung in Gegenwart üblicher Hydrierungskatalysatoren (Edelmetallkatalysatoren), insbesondere Palladium-Katalysatoren oder auch Platinkatalysatoren (Platinoxyd), Raney-Nickel, in einem Lösungs-oder Suspensionsmittel, gegebenenfalls unter erhöhtem Druck, (zum Beispiel 1 -50 bar) bei Temperaturen zwischen 20 - 150° C, insbesondere 30 -100° C, vorzugsweise 40 -80° C abgespalten.

Als Lösungs-beziehungsweise Suspensionsmittel für die Abspaltung solcher Schutzgruppen kommen beispielsweise in Betracht: Wasser, niedere aliphatische Alkohole, cyclische Ether wie Dioxan oder Tetrahydrofuran, aliphatische Ether, Halogenkohlenwasserstoffe, Dimethylformamid und so weiter sowie Mischungen dieser Mittel. Als Schutzgruppen, die durch Hydrogenolyse abspaltbar sind, kommen beispielsweise in Frage: Benzylrest, α-Phenyläthylrest, im Benzolkern substituierte Benzylreste (p-Brom-oder p-Nitrobenzylrest), Carbobenzoxyrest, Carbobenzthiorest, tert.-Butyloxycarbonylrest. Beispiele für hydrolytische abspaltbare Reste sind: Trifluoracetylrest, Phthalylrest, Tritylrest, p-Toluolsulfonylrest, tert.-Butyloxycarbonylrest, tert.-Butylrest, Dimethylmethylenrest und ähnliche sowie niedere Alkanoylreste wie Acetylrest, Formylrest, tert.-Butylcarboxyrest und ähnliche.

Insbesondere kommen die bei der Peptid-Synthese üblichen Schutzgruppen und die dort üblichen Abspaltungsverfahren in Frage. Unter anderem wird hierzu auch auf das Buch von Jesse P. Greenstein und Milton Winitz "Chemistry of Amino Acids", New York 1961, John Wiley and Sons, Inc. Volume 2, beispielsweise Seite 883 und folgende, verwiesen. Auch die Carbalkoxygruppe (zum Beispiel niedrigmolekulare) kommt in Frage.

Die Abspaltung der Gruppe OZ (vorzugsweise ist diese $OCH_3$) erfolgt beispielsweise mit Alkalibromiden oder Alkalijodiden, niederen Alkylmagnesiumhalogeniden oder mit primären, sekundären oder tertiären Aminen, insbesondere den entsprechenden niederen Alkylaminen, wie zum Beispiel tertiären $C_1$-$C_6$-Alkylaminen (Trimethylamin). Als Alkalibromide beziehungsweise Alkalijodide kommen zum Beispiel in Frage: Lithiumbromid, Natriumbromid, Lithiumjodid, Natriumjodid. Als niedere Alkylmagnesiumhalogenide kommen zum Beispiel in Frage: Methylmagnesiumjodid, Methylmagnesiumbromid (Lösungsmittel in diesem Fall niedere aliphatische Ether, wie Diethylether).

Die Abspaltung der Gruppe OZ aus einer Verbindung der Formel III erfolgt bei Temperaturen zwischen 10 und 150° C, vorzugsweise 10 und 80° C, insbesondere 50 und 80° C, wobei man das bis dahin erhaltene Reaktionsprodukt nach Entfernung der Lösungsmittel in einem inerten Mittel auflöst. Als solche inerten Mittel kommen in Frage: Gesättigte aliphatische $C_3$-$C_8$-Ketone (Ethylmethylketon, Diethylketon, Aceton), cyclische Ether, nichtcyclische niedere aliphatische Ether (zum Beispiel Diethylether). Pro 1 Mol der eingesetzten Verbindung III werden im allgemeinen 1,5 bis 3 Mol der zuvor genannten Absoaltungsmittel, vorzugsweise 2 Mol verwendet.

Die Umsetzung von erhaltenen Produkten (beispielsweise Verbindungen, bei welchen $R^1$ und/oder $R^2$ Halogenalkyl bedeuten) mit Ammoniak oder einem Amin der Formel $NR^3R^4R^5$ erfolgt bei Temperaturen zwischen 10 und 200, vorzugsweise 20 und 150° C, insbesondere 40 und 80° C mit oder ohne Lösungsmittel. Falls ein Lösungs -oder Suspensionsmittel verwendet wird, kommen hierfür in Frage: Aromatische Kohlenwasserstoffe, wie zum Beispiel Pentan, Hexan, Heptan, Benzol, Mesitylen, Toluol, Xylol; niedere aliphatische Ketone wie zum Beispiel Aceton, Methyläthylketon; halogenierte Kohlenwasserstoffe, wie zum Beispiel Chloroform, Trichlorethylen, Tetrachlorkohlenstoff, Chlorbenzol, Methylenchlorid; cyclische Ether wie zum Beispiel Tetrahydrofuran und Dioxan; niedere aliphatische nichtcyclische Ether (Diethylether, Diisopropylether); niedere aliphatische Alkohole (1 -6 C-Atome), wie zum Beispiel Methanol, Äthanol, Isopropanol, Amylalkohol, Butanol, tert.-Butanol; Amide und N-alkyl-substituierte Amide von aliphatischen $C_1$-$C_4$-Carbonsäuren (Dimethylformamid, Dimethylacetamid); $C_1$-$C_6$-Dialkylsulfone (Dimethylsulfon, Tetramethylsulfon); $C_1$-$C_6$-Dialkylsulfoxide (Dimethylsulfoxid) sowie weitere aprotische Mittel wie N-Methylpyrrolidon, Tetramethylharnstoff, Hexamethylphosphorsäuretriamid, Acetonitril. Die einzelnen Alkylreste der oben angegebenen Lösungsmittel enthalten beispielsweise 1 -6, insbesondere 1 -4 Kohlenstoffatome. Auch Gemische dieser Mittel sowie Mischungen mit Wasser kommen als Reaktionsmedium in Frage. Die Reaktion wird beispielsweise bei Temperaturen von 0 bis 200° C, vorzugsweise 20 bis 150° C oder auch 50 bis 120° C durchgeführt. Wird ein Lösungs-beziehungsweise Dispersionsmittel verwendet, arbeitet man häufig bei der Rückflußtemperatur dieses Mittels.

Diese Aminisierungsreaktion wird zweckmäßig in Gegenwart basischer Stoffe durchgeführt. Als basische Stoffe kommen beispielsweise in Frage: Alkalihydroxyde, Alkalicarbonate, tertiäre Amine.

Die Alkylierung von freien Aminogruppen in den Resten $R^1$ und/oder $R^2$ erfolgt bei Temperaturen zwischen 0 und 200, vorzugsweise zwischen 20 und 150, insbescndere 20 und 80° C. Diese Alkylierung erfolgt beispielsweise durch Umsetzung mit Verbindungen der Formel R'Hal, $ArSO_2OR'$ und $SO_2(OR'_3)_2$, wobei Hal ein Halogenatom (insbesondere Chlor, Brom oder Jod) und Ar ein aromatischer Rest (zum Beispiel ein gegebenenfalls durch einen oder mehrere niedere Alkylreste substituierter Phenyl-oder Naphthylrest und R' eine $C_1$-$C_6$-Alkylgruppe ist. Beispiele sind p-Toluolsulfonsäure-$C_1$-$C_6$-alkylester, $C_1$-$C_6$-Dialkylsulfate, $C_1$-$C_6$-Alkylhalogenide. Die Alkylierungsreaktion wird gegebenenfalls unter Zusatz von iblichen säurebindenen Mitteln, wie Alkalihydroxiden, Alkalicarbonaten, Alkalihydrogencarbonaten, Erdalkalicarbonaten, Alkaliacetaten, tertiären Aminen (zum Beispiel Trialkylamin wie Triethylamin), Pyridin oder auch Alkalihydriden in inerten Lösungsmitteln oder Suspensionsmitteln durchgeführt. Als Lösungs-oder Dispergiermittel kommen beispielsweise in Betracht: Aromatische Kohlenwasserstoffe wie zum Beispiel Benzol, Toluol, Xylol; aliphatische Ketone wie zum Beispiel Aceton, Methylethylketon; halogenierte Kohlenwasserstoffe wie zum Beispiel Chloroform, Tetrachlorkohlenstoff, Chlorbenzol, Methylenchlorid; aliphatische Ether wie zum Beispiel Butylether; cyclische Ether wie zum Beispiel Tetrahydrofuran, Dioxan; Sulfoxyde wie zum Beispiel Dimethylsulfoxyd; tertiäre Säureamide wie zum Beispiel Dimethylformamid, N-Methylpyrrolidon, Hexamethylphosphorsäuretriamid; aliphatische Alkohole wie Methanol, Ethanol, Isopropanol, Amylalkohol, tert.-Butanol, cycloaliphatische Kohlenwasserstoffe wie Cyclohexan und ähnliche. Auch wässrige Mischungen der genannten Lösungsmittel können verwendet werden. Häufig arbeitet man bei der Rückflußtemperatur der verwendeten Lösungs-beziehungsweise Dispergiermittel. Häufig werden die Alkylierungsreaktionskomponenten im Überschuß eingesetzt. Die Alkylierung kann auch in Gegenwart von Tetraalkylammoniumsalzen - (insbesondere der Halogenide) in Kombination mit Alkalihydroxiden bei Temperaturen zwischen 0 -100° C, vorzugsweise 20 -80° C in einem aprotischen Lösungsmittel oder auch in Chloroform oder Methylenchlorid vorgenommen werden. Als aprotische Lösungsmittel kommen insbesondere in Betracht: tertiäre Amide - (Dimethylformamid, N-Methyl-Pyrrolidon, Hexamethylphosphorsäuretriamid), Dimethylsulfoxid, Acetonitril, Dimethoxyethan, Aceton, Tetrahydrofuran.

Die im erfindungsgemäßen Arzneimittel enthaltenen als Wirkstoffe der allgemeinen Formel I beziehungsweise I' sind teilweise neu und insoweit ebenfalls ein Gegenstand der Erfindung. Sie besitzen eine ausgeprägte cytotoxische Wirksamkeit, die sowohl in vivo am chemisch induzierten Mamma-Karzinom der Ratte, als auch in vitro an Leukämiezellen in der Zellkultur nachgewiesen wurde. Darüberhinaus wurden in einer klinischen Pilotstudie bei Patientinnen mit Mamma-Karzinom Hautmetastasen zur vollständigen Abheilung bei topischer Anwendung gebracht.

Die folgenden Verbindungen und deren physiologisch verträgliche Salze sind neu:

Verbindungen der allgemeinen Formel

$R-Y-PO_2^{\ominus}-X-R_1$ I'

worin R einen gesättigten oder ungesättigten Kohlenwasserstoffrest mit 12 bis 24 C-Atomen bedeutet, der auch halogensubstituiert sein kann,

X ein Sauerstoffatom, NH oder $NR_2$ und Y ein Sauerstoffatom oder NH ist,

der Rest $R_1$

a) eine $C_1$-$C_8$-Alkylgruppe, eine ungesättigte $C_3$-$C_8$-Alkylgruppe oder eine gegebenenfalls ungesättigte $C_3$-$C_8$-Alkylgruppe, die durch Halogen, Amino, $C_1$-$C_6$-Alkylamino, Di-$C_1$-$C_6$ alkylamino, Tri-$C_1$-$C_6$-alkylamino, Hydroxy, Carboxy, $C_3$-$C_8$-Cycloalkyl oder Phenyl substituiert ist, darstellt oder

b) eine $C_2$-Alkylgruppe, die durch Halogen, Hydroxy, Carboxy, $C_3$-$C_8$-Cycloalkyl oder Phenyl substituiert ist, darstellt, oder

c) eine ungesättigte $C_2$-Alkylgruppe, die durch Di-$C_1$-$C_6$-alkylamino, Tri-$C_1$-$C_6$-alkylamino, Carboxy, $C_3$-$C_8$-Cycloalkyl oder Phenyl substituiert ist, darstellt, oder

d) eine $C_2$-Alkylgruppe, die durch Amino, $C_1$-$C_6$-Alkylamino, Di-$C_1$-$C_6$-alkylamino oder Tri-$C_1$-$C_6$-alkylamino substituiert ist, darstellt, falls X Sauerstoff, NH oder $NR_2$ und Y die Gruppe NH bedeutet oder falls X die Gruppe NH oder $NR_2$ und Y Sauerstoff bedeutet und R die angegebenen Bedeutungen hat, oder

e) 2-tert.-Butyloxycarbonylaminoethyl,

2-tert.-Butyloxycarbonylethyl, 2,3-Isopropylidendioxy-propyl-(1), 2,3-Dibenzyloxypropyl-(1), 1,3-Dibenzyloxypropyl-(2) oder N-$C_1$-$C_6$-Alkylamino-$C_2$-$C_6$-alkyl bedeuten kann, wenn X ein Sauerstoffatom ist und Y und R die angegebenen Bedeutungen haben,

oder

f) 2,3-Dihydroxypropyl-(1) bedeuten kann, wenn X die NH-Gruppe ist und Y und R die angegebenen Bedeutungen haben, und $R_2$ eine 2,3-Dihydroxypropyl-(1)-gruppe, eine $C_1$-$C_8$-Alkylgruppe oder eine $C_2$-$C_8$-Alkylgruppe, die ungesättigt und/oder mit Halogen, Amino, $C_1$-$C_6$-Alkylamino, Di-$C_1$-$C_6$-alkylamino, Tri-$C_1$-$C_6$-alkylamino, Hydroxy, Carboxy, $C_3$-$C_8$-Cycloalkyl oder Phenyl substituiert ist, darstellt,

wobei solche Verbindungen ausgenommen sind, wo in der Formel I' X und Y beide Sauerstoff sind, $R_1$ ein gesättigter oder ungesättigter $C_1$-$C_8$-Alkylrest ist, der auch durch Hydroxy, Amino, $C_1$-$C_6$-Alkylamino, Di-$C_1$-$C_6$-alkylamino oder Tri-$C_1$-$C_6$-alkylamino substituiert sein kann und R einen gesättigten oder ungesättigten $C_{12}$-$C_{24}$-Alkylrest darstellt.

Insbesondere für die topische Applikation, aber auch für die Zubereitung als Arzneimittel für andere Applikationsarten hat es sich als besonders günstig herausgestellt, die Verbindungen der allgemeinen Formel I beziehungsweise I′ zusammen mit wenigstens einem Alkylglycerin mit 3 bis 12 Kohlenstoffatomen im Alkylrest, der in Form einer Ethergruppe an eine der primären oder sekundären OH-Gruppen des Glycerins gebunden vorliegen kann, einzusetzen. Derartige Alkylglycerine steigern beziehungsweise verbessern die Wirkung der Verbindungen der allgemeinen Formel I beziehungsweise I′ synergistisch. Bevorzugt werden hierbei Alkylglycerine mit 3 bis 9 C-Atomen allein oder in Mischung verwendet.

Besonders günstige Wirkungen besitzt daher ein synergistisch wirkendes Arzneimittel, welches

a) mindestens eine Verbindung der allgemeinen Formel

$R-Y-PO_2^{\ominus}-X-R_1$   I

oder ein physiologisch verträgliches Salz hiervon enthält, wobei in der Formel I
R einen gesättigten oder ungesättigten Kohlenwasserstoffrest mit 12 bis 24 C-Atomen bedeutet, der auch halogensubstituiert sein kann,
X ein Sauerstoffatom, NH oder $NR_2$ und Y ein Sauerstoffatom oder NH ist, $R_1$ eine $C_1$-$C_8$-Alkylgruppe ist, oder worin $R_1$ eine $C_2$-$C_8$-Alkylgruppe darstellt, die ungesättigt und/oder mit Halogen, Amino, $C_1$-$C_6$-Alkylamino, Di-$C_1$-$C_6$-alkylamino, Tri-$C_1$-$C_6$-alkylamino, Hydroxy, Carboxy, $C_3$-$C_8$-Cycloalkyl oder Phenyl substituiert ist, und worin $R_1$ außerdem auch 2-tert.-Butyloxycarbonylaminoethyl, 2-tert.-butyloxycarbonylethyl, 2,3-Isopropylidendioxy-propyl-(1), 2,3-Dibenzyloxy-propyl-(1), 1,3-Dibenzyloxy-propyl-(2) oder N-$C_1$-$C_6$-Alkylamino$C_2$-$C_6$-alkyl bedeuten kann, wenn X ein Sauerstoffatom ist, und worin $R_1$ außerdem auch 2,3-Dihydroxypropyl-(1) bedeuten kann, wenn X die NH-Gruppe ist,
und $R_2$ eine 2,3-Dihydroxypropyl-(1)-gruppe, eine $C_1$-$C_8$-Alkylgruppe oder eine $C_2$-$C_8$-Alkylgruppe, die ungesättigt und/oder mit Halogen, Amino, $C_1$-$C_6$-Alkylamino, Di-$C_1$-$C_6$ alkylamino, Tri-$C_1$-$C_6$-alkylamino, Hydroxy, Carboxy, $C_3$-$C_8$-Cycloalkyl oder Phenyl substituiert ist, darstellt, und

b) ein Alkylglycerin der allgemeinen Formel II

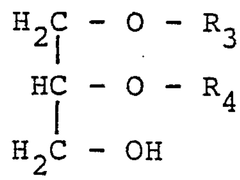

in der einer der Reste $R_3$ und $R_4$ eine Alkylgruppe mit 3 bis 12 C-Atomen und der andere Rest ein H-Atom bedeutet, enthält,
sowie gegebenenfalls weitere übliche pharmakologische Zusatz-und Verdünnungsmittel.

Eine derartige Mischung wird im folgenden auch als Kaskade bezeichnet.
Der Gehalt an Verbindung der allgemeinen Formel I beziehungsweise I′ in mg/ml Kaskade wird durch einen nachgesetzten Index bezeichnet, derart, daß zum Beispiel ein Kaskadengemisch, welches 5 mg/ml Verbindung von Formel I beziehungsweise I′ enthält, als $Kaskade_5$, ein Gemisch mit 200 mg Verbindung der Formel I beziehungsweise I′ pro ml Kaskade als $Kaskade_{200}$ bezeichnet wird.

Die Herstellung der Alkylglycerine ist bekannt, beispielsweise aus der DE-OS 33 43 530.8.
Beispielsweise werden Alkylglycerin-Wasser-Mischungen, welche zum Beispiel Nonylglycerin, Octylglycerin, Hexylglycerin, Pentylglycerin, Propylglycerin und Ethylglycerin enthalten, bevorzugt. Vorzugsweise enthalten solche wäßrigen Mischungen 3 der genannten Glycerinether und zwar einen niederen (Ethyl, Propyl), einen mittleren (Petnyl, Hexyl) und einen höheren (Nonyl, Octyl), wobei die Gewichtsmenge an dem niederen Ether etwa so groß ist wie die Summe der Gewichtsmengen an den beiden anderen Glycerinethern. Die Wassermenge ist etwa gleich der Menge an dem niederen Glycerinether, und beträgt beispielsweise die Hälfte der Gesamtmenge an den vorliegenden Glycerinethern. Beispiele für solche Glycerinether-Wasser-Mischungen sind im folgenden angeführt:

| | Wasser | | Glycerin-Propylether | | Glycerin-Hexylether | | Glycerin-Nonylether |
|---|---|---|---|---|---|---|---|
| Gewichtsteile | 2 | : | 2 | : | 1 | : | 1 |

| | Wasser | | Glycerin-Ethylether | | Glycerin-Pentylether | | Glycerin-Octylether |
|---|---|---|---|---|---|---|---|
| Gewichtsteile | 2 | : | 2 | : | 1 | : | 1 |

Zur topischen Applikation sind die erfindungsgemäßen Arzneimittel in besonderem Maße geeignet. Um Hauttumoren beziehungsweise Hautmetastasen mit diesem Arzneimittel zu behandeln, werden die betroffenen Hautbezirke beispielsweise mit Kaskade$_5$ bis Kaskade$_{200}$ zwei-bis dreimal täglich eingerieben. Schädliche Nebenwirkungen konnten bisher nicht beobachtet werden, auch nicht bei Patienten, die über einen Zeitraum von 3 Monaten behandelt wurden. Die Remission der Hautmetastasen ist begleitet von einer Normalisierung der Haut, wie durch Gewebeschnitte eindeutig nachgewiesen werden konnte. Mehrere Patientinnen mit Hautmetastasen wurden auf diese Weise behandelt und hierbei ein vollständiges Verschwinden der Mamma-Karzinom-Hautmetastasen beobachtet.

Die topische Behandlung mit dem erfindungsgemäß bevorzugten Mittel in der Formulierung Kaskade$_5$ bis Kaskade$_{200}$ läßt sich auch für die Behandlung von inneren Tumoren bzw. Metastasen durch großflächiges Einreiben der Haut anwenden. Über die Resorption durch die Haut werden hierbei therapeutisch wirksame Blutspiegel erreicht. Ein Vorteil dieser Applikationsart liegt darin, daß die Zubereitungen Kaskade$_5$ bis Kaskade$_{200}$ von der Haut problemlos toleriert werden.

Diese bevorzugte Zubereitungsart des erfindungsgemäßen Arzneimittels in Form der Lösungen Kaskade$_5$ bis Kaskade$_{200}$ eignet sich auch gut für die Herstellung von Suppositorien für die rektale Einführung. Auch hiermit lassen sich innere Tumoren bzw. innere Metastasen gut behandeln.

Eine andere Anwendungsart des erfindungsgemaßen Arzneimittels besteht in der Instillation in präformierte Körperhöhlen. Diese Anwendungsart eignet sich besonders für Pleurakarzinosen, malignen Aszites, maligne Perikardergüsse und Blasenkarzinome. In diesem Fall werden die erfindungsgemäßen Antitumormittel der allgemeinen Formel I entweder alleine oder in Verbindung mit üblichen Träger-und Verdünnungsmitteln, insbesondere auch mit Kaskade eingesetzt werden.

Zur systematischen Applikation kommt orale oder intravenöse Verabreichung in Betracht.

Für die orale Verabreichung werden die Verbindungen der allgemeinen Formel I zweckmäßig in Form einer Trinklösung angewendet. Als Träger eignen sich beispielsweise Milch, Kakao, Fruchtsaft oder Trinkwasser. Die Herstellung einer solchen Trinklösung kann zum Beispiel durch Verdünnen einer konzentrierten alkoholischen Lösung einer Verbindung der Formel I beziehungsweise I′ gemäß den Ansprüchen 1 und/oder 2 mit Wasser oder einem anderen der zuvor genannten Mittel erfolgen. Bei Ratten führten Tagesdosen von 20, 40 und 60 mg/kg Körpergewicht bei Verwendung von Hexadexylphosphocholin und Oleylphosphocholin zu einer vollständigen Remission von chemisch induzierten Mamma-Karzinomen. Hierbei erwiesen sich diese Verbindungen als besser wirksam und besser verträglich als 1-Octadecyl-2-methyl-rac-glycero-3-phosphochlolin. Bei dem für diese Versuche verwendeten Tumormodell handelt es sich um ein sogenanntes hartes Modell. Dies bedeutet, daß die an diesem Modell erstellten Befunde auch auf die humane Situation übertragbar sind.

Für die intravenöse Verabreichung über die intravenöse Infusionstherapie werden die Verbindungen der Formel I beziehungsweise I′ zweckmäßig in physiologischer Kochsalzlösung angewendet. Auch andere Infusionslösungen können hierbei angewendet werden. Dosis am Menschen für solche Lösungen ist beispielsweise 1 -10 mg/kg Körpergewicht.

Schließlich können mehrere Applikationsarten des erfindungsgemäßen Arzneimittels kombiniert angewendet werden, wobei die besondere topische Verträglichkeit dazu führt, daß einerseits ein Einreiben der Haut mit einer der anderen Applikationsformen kombiniert angewendet wird.

Eine weitere Trägermischung für die Verbindungen der Formel I beziehungsweise I′, die sich besonders bewährt hat, besteht aus einer Mischung von etwa 4 Gewichtsteilen Wasser, 4 Gewichtsteilen Propylglycerin und je 2 Gewichtsteilen Hexylglycerin und Nonylglycerin.

Die topische Anwendung des erfindungsgemäßen Arzneimittels in der besonders bevorzugten Zubereitsungsform Kaskade$_5$ bis Kaskade$_{200}$ über einen Zeitraum von mehreren Monaten zeigte, daß die lokale Toxizitat sich auf ein verstärktes Abschuppen der Haut, ähnlich wie bei der lokalen Anwendung von Acetylsalicylsäure, beschränkt.

Die Erfindung stellt somit ein neues Arzneimittel zur Behandlung von Tumoren zur Verfügung und liefert hierbei nicht nur überhaupt ein weiteres Antitumormittel, sondern bringt erstmals ein auch bei topischer Anwendung im klinischen Versuch nachgewiesenermaßen wirksames Mittel. Hierdurch werden für die Behandlung von Tumorpatienten neue Möglichkeiten eröffnet.

Zur Herstellung von entsprechenden Arzneimitteln wird mindestens eine Verbindung der Formel I beziehungsweise I' mit gebräuchlichen pharmazeutischen Trägerstoffen und/oder Verdünnungsmitteln beziehungsweise sonstigen Hilfsstoffen zu pharmazeutischen Zubereitungen verarbeitet beziehungsweise in eine therapeutisch anwendbare Form gebracht. Dies erfolgt zum Beispiel dadurch, daß man Verbindungen der Formel I beziehungsweise I' , worin die einzelnen Reste und Symbole die angegebenen Bedeutungen haben, beziehungsweise deren physiologisch verträgliche Salze zusammen mit üblichen Träger-und/oder Verdünnungsbeziehungsweise Hilfsstoffen bei Temperaturen zwischen 20 und 120° C, vorzugsweise 30 - 100° C vermischt beziehungsweise homogenisiert, die so erhaltene Mischung zur Herstellung von Zubereitungen, die in der Dosierungseinheit 5 bis 2000 mg, vorzugsweise 10 bis 500 mg, insbesondere 30 bis 400 mg Wirkstoff der Formel I beziehungsweise I' enthalten, in Hohlzellen entsprechender Größe ausgießt oder in Kapseln entsprechender Größe abfüllt oder granuliert und dann, gegebenenfalls unter Zusatz von weiteren üblichen Hilfsstoffen zu Tabletten verpreßt.
Zum Beispiel dadurch, daß man Verbindungen der Formel I beziehungsweise I' mit einem oder mehreren der folgenden Stoffe: Stärke, Cellulose, Lactose, Formalin-Casein, modifizierte Stärke, Magnesiumstearat, Calciumhydrogen phosphat, hochdisperse Kieselsäure, Talkum, Phenoxyethanol vermischt, die erhaltene Mischung, gegebenenfalls mit einer wässrigen Lösung, die als Bestandteil mindestens Gelatine, Stärke, Polyvinylpyrrolidon, VinylpyrrolidonVinylacetat-Copolymerisat und/oder Polyoxyethylsorbitanmonooleat enthält, granuliert, das Granulat gegebenenfalls mit einem oder mehreren der obengenannten Hilfsstoffe homogenisiert, und diese Mischung zu Tabletten verpresst oder in Kapseln abfüllt, wobei solche Tabletten oder Kapseln in der Dosierungseinheit jeweils 5 bis 2000 mg Wirkstoff der Formel I beziehungsweise I' enthalten; oder daß man Verbindungen der Formel I oder deren Salze nach Zusatz von Sojalecithin sowie gegebenenfalls 0,1 -0,5 Gewichtsteilen Phenoxyethanol (bezogen auf einen Gewichtsteil Verbindung I beziehungsweise I') bei Temperaturen zwischen 33 -37° C in geschmolzenem Hartfett suspendiert und homogenisiert und anschließend die Mischung in Hohlzellen ausgießt,wobei die Dosierungseinheit 5 bis 2000 mg Wirkstoff sowie gegebenenfalls 0,1 -0,5 Gewichtsteile Phenoxyethanol (bezogen auf einen Gewichtsteil Verbindung I oder I') enthält; oder daß man Verbindungen der Formel I beziehungsweise I' oder deren Salze bei einer Temperatur zwischen 50 bis 120° C, vorzugsweise 50 bis 100° C, gegebenenfalls in Gegenwart eines oder mehrerer Emulgatoren und/oder 0,1 -0,5 Gewichtsteilen Phenoxyethanol (bezogen auf einen Gewichtsteil Verbindung I oder I') mit mindestens einem der folgenden Stoffe homogenisiert: Paraffin, Vaseline, aliphatischer Alkohol mit 12 bis 25 C-Atomen, aliphatische Monocarbonsäure mit 15 bis 20 C-Atomen, Sorbitanmonopalmitat, Polyoxyethylenpolyolfettsäureester, und die erhaltene Mischung zwischen 50 und 120° C mit Wasser, gegebenenfalls unter Zusatz eines mehrwertigen niederen aliphatischen Alkohols und/oder Phenoxyethanol emulgiert; oder daß man Verbindungen der Formel I beziehungsweise I' oder deren Salze in Wasser oder Pflanzenöl, gegebenenfalls in Gegenwart von 0,1 -0,5 Gewichtsteilen Phenoxyethanol (bezogen auf einen Gewichtsteil Verbindung I oder I') sowie gegebenenfalls in Gegenwart eines Emulgators, bei Temperaturen zwischen 30 -100° C auflöst, und gegebenenfalls die so erhaltene Lösung mit soviel Wasser oder Pflanzenöl auffüllt, daß die Endlösung 0,05 bis 10 Gewichtsprozent, vorzugsweise 0,1 bis 5 Gewichtsprozent an Wirkstoff der Formel I beziehungsweise I' enthält.

Als Emulgatoren kommen zum Beispiel in Frage: nichtionogene Emulgatoren sowie ionogene Emulgatoren. Bei den nichtionogenen Emulgatoren handelt es sich beispielsweise um Triglyceridgemische von gesättigten Pflanzenfettsäuren mit $C_8$, $C_{10}$ und $C_{12}$ oder um Emulgatoren auf der Basis von Polyadditionsprodukten des Ethylenoxids, wie zum Beispiel alkyl-und acylsubstituierte Polyadditionsprodukte des Ethylenoxids, Polyethylenglykol-fettsäureester, Umsetzungsprodukte von Ethylenoxid mit Ricinusöl beziehungsweise hydriertem Ricinusöl, Ester von hydrierten Ricinusölfettsäuren mit oxyethyliertem Glycerin. Weiterhin kann es sich um Emulgatoren auf Basis von Fettsäureamiden oder Fettsäurekondensationsprodukten mit hydrophilen Gruppen handeln. Als ionogene Emulgatoren kommen zum Beispiel Emulgatoren auf Basis von Fett säuremonoestern des Glycerins oder anderer mehrwertiger Alkohole (Lunacera alba) in Frage.

Falls bei der wie oben angegebenen Herstellung der Arzneimittel der oder die Wirkstoffe der Formel I beziehungsweise I' in Gegenwart von einem Glycerinether der Formel II oder einer Mischung von solchen Glycerinethern der Formel II verwendet werden, wird eine synergistische Wirkungssteigerung der Antitumorwirkung beobachtet.
Hierzu werden die Wirkstoffe der Formel I beziehungsweise I' mit 1 bis 30, vorzugsweise 2 bis 20 Gewichtsteilen (bezogen jeweils auf einen Gewichtsteil an Verbindung I oder I') von mindestens einem

Glycerinether der Formel II oder einem Gemisch solcher Glycerinether sowie gegebenenfalls 0,5 -30, vorzugsweise 1 -20 Gewichtsteilen Wasser (ebenfalls bezogen auf einen Gewichtsteil an Verbindung I beziehungsweise I') verwendet. Diese Vermischung mit den Glycerinethern kann bei der Herstellung der entsprechenden Arzneimittel am Anfang erfolgen, aber gegebenenfalls auch in einem späteren Herstellungsstadium.

Die erfindungsgemäßen Verbindungen der Formeln I und I' zeigen beispielsweise eine gute Wirkung am 7,12-Dimethylbenzanthracen induzierten Brustdrüsenkrebs der Ratte; ebenso am Methylnitrosoharnstoff-induzierten Mammacarcinom der Ratte.

Beispielsweise wird bei obengenannter Versuchsmethode bei einer Dosis von 10 mg/kg Körpergewicht Ratte ein Wachstumsstillstand der Tumore, bei höheren Dosen auch ein völliges Verschwinden der Geschwülste erzielt.

Die niedrigste, bereits wirksame Dosis in dem obenangegebenen Tierversuch ist beispielsweise
5 mg/kg oral
5 mg/kg intravenös.

Als allgemeiner Dosisbereich für die Wirkung (Tierversuch wie oben) kommt beispielsweise in Frage:
5 -50 mg/kg oral, insbesondere 15 -32 mg/kg
5 -50 mg/kg intravenös, insbesondere 15 -32 mg/kg.

Die Wirkungsrichtung der erfindungsgemäßen Verbindungen ist mit der Wirkung des bekannten Arzneimittelwirkstoffs TAMOXIFEN vergleichbar, jedoch bestehen hierzu insbesondere folgende Unterschiede: Die Wirkung ist stärker und von längerer Dauer als die von TAMOXIFEN.

Indikationen, für die die erfindungsgemäßen Verbindungen in Betracht kommen können: Brustdrüsenkrebs und andere menschliche Krebsarten.

Die pharmazeutischen Zubereitungen enthalten im allgemeinen zwischen 5 -2000 mg, beispielsweise 10 - 400 mg der erfindungsgemäßen aktiven Komponenten.

Die Verabreichung kann beispielsweise in Form von Tabletten, Kapseln, Pillen, Dragees, Zäpfchen, Salben, Gelees, Cremes, Puder, Stäubepulver, Aerosolen oder in flüssiger Form erfolgen. Als flüssige Anwendungsformen kommen zum Beispiel in Frage: Ölige oder alkoholische beziehungsweise wässrige Lösungen sowie Suspensionen und Emulsionen. Bevorzugte Anwendungsformen sind Tabletten, die zwischen 40 und 400 mg oder Lösungen, die zwischen 0,1 % bis 5 % an aktiver Substanz enthalten.

Die Einzeldosis der erfindungsgemäßen aktiven Komponenten kann beispielsweise liegen

a) bei oralen Arzneiformen zwischen 5 -100 mg/kg Körpergewicht, vorzugsweise 15 -50 mg/kg Körpergewicht,

b) bei parenteralen Arzneiformen (zum Beispiel intravenös, intramuskulär) zwischen 5 -100 mg/kg Körpergewicht,

c) bei Arzneiformen zur lokalen Applikation auf die Haut und Schleimhäute (zum Beispiel in Form von Lösungen, Lotionen, Emulsionen, Salben und so weiter) zwischen 50 -2000 mg, vorzugsweise 80 -1500 mg.

-(Die Dosen sind jeweils bezogen auf die freie Base) -

Beispielsweise können 3 mal täglich 1 Tablette mit einem Gehalt von 40 -400 mg wirksamer Substanz oder zum Beispiel bei intravenöser Injektion 1 -5 mal täglich eine Ampulle von 1 -5 ml Inhalt mit 50 -250 mg Substanz empfohlen werden. Bei oraler Verabreichung ist die minimale tägliche Dosis beispielsweise 120 mg; die maximale tägliche Dosis bei oraler Verabreichung soll nicht über 100 mg/kg Körpergewicht liegen.

Die akute Toxizität der erfindungsgemäßen Verbindungen an der Maus (ausgedrückt durch die LD 50 mg/kg; Methode nach Miller und Tainter: Proc. Soc. Exper. Biol. a. Med. 57 (1944) 261) liegt beispielsweise bei oraler Applikation zwischen 200 und 450 mg/kg Körpergewicht.

Die Arzneimittel können in der Humanmedizin, der Veterinärmedizin sowie in der Landwirtschaft allein oder im Gemisch mit anderen pharmakologisch aktiven Stoffen verwendet werden.

Die Erfindung wird durch die folgenden Beispiele erläutert.

**Beispiel 1**

Hexadecylphosphoethanolamin (Phosphorylierung, Ringschluß und Ringöffnung)

Hexadecanol (1 Mol, 243 g) und Triethylamin (1,8 Mol, 180 g) werden in 1,5 l THF (Tetrahydrofuran) gelöst und tropfenweise zu einer stark gerührten Lösung von Phosphoroxychlorid (1,2 Mol, 184 g) in 120 ml THF so zugegeben, daß die Temperatur im Reaktionsgefäß (Dreihals, 5 l, mit Tropftrichter, Thermometer und Rührer) 10°C nicht übersteigt. Zur Beschleunigung des Vorgangs wird das Reaktionsgefäß mit einer Eis-Kochsalzmischung gekühlt. Unmittelbar nach dem Eintropfen ist die Reaktion abgeschlossen (Nachweis über DSC in Ether: Rf-Werte von 0,8 für das Ausgangsprodukt, von 0,0 für das Reaktionsprodukt nach Hydrolyse mit Wasser).

Man entfernt das Eisbad und tropft in das Reaktionsgemisch unter starkem Rühren eine Lösung von Ethanolamin (1,5 Mol, 92 g) und Triethylamin (1,8 Mol, 180 g) in 1 l Dioxan so ein, daß die Temperatur im Reaktionsgefäß auf 65 bis 70°C steigt. Dann ist die Ringbildung abgeschlossen (Nachweis durch DSC in Ether: Rf-Wert von 0,2). Man filtriert von ausgefallenem Triethylaminhydrochlorid noch warm ab und versetzt das Filtrat bei 40 bis 50°C mit 1,5 l 2N Ameisensäure. Nach 15 Minuten ist die Ringöffnung abgeschlossen (Nachweis durch DSC in Ether Rf-Wert 0,0; DSC in Chloroform/Methanol/Essigsäure/Wasser 100:60:20:5 per Vol.:Rf-Wert 0,8). Man kühlt auf -20°C und filtriert vom Niederschlag ab, der aus weitgehend reinem Hexadecylphosphoethanolamin besteht. Bei leichten Verunreinigungen wird eine chromatographische Reinigung angeschlossen (siehe Beispiel 2). Mikroanalyse (MG 365,50):
ber. (%): C 59,15 H 11,03 N 3,83 P 8,48
gef. (%): 59,01 10,95 3,79 8,31

**Beispiel 2**

Hexadecylphosphocholin * 1 $H_2O$ (Methylierung von I)

Die nach Beispiel 1 erhaltenen Kristalle werden ohne weitere Reinigung in 1,2 l 2-Propanol und 0,4 l Dichlormethan aufgenommen. Man versetzt die Suspension der Kristalle unter starkem Rühren mit Kaliumkarbonat (4 Mol, 560 g) in 1 l Wasser. Das zweiphasige Reaktionsgemisch wird mit Dimethylsulfat (4 Mol, 500 g) tropfenweise und unter Rühren so versetzt, daß die Temperatur 40°C nicht übersteigt. Die Reaktion ist 60 Minuten nach dem Eintropfen beendet (Nachweis durch DSC in Chloroform/Methanol/25%igem Ammoniak 50:50:5 per Vol.: Rf-Wert 0,3). Nach Phasenseparation bei 20°C enthält die obere Phase das Produkt. Man entfernt das Lösungsmittel am Rotationsverdampfer unter Vakuum und chromatographiert den viskosen Rückstand an Kieselgel (Merck Art. 7733, Kieselgel 60, Korngröße 0,2 bis 0,5 mm).

Chromatographie

Kieselgel, 2 kg, werden mit Chloroform/Methanol/25%igem Ammoniak (200/15/1 per Vol.) versetzt und in eine Chromatographiesäule gefüllt. Man löst das viskose Öl in 800 ml des obigen Lösungsmittelgemisches und gibt das Rohprodukt auf die Säule (unlösliche Anteile werden vorher abfiltriert). Man eluiert mit Fließmitteln steigender Polarität bis die Verunreinigungen ausgewaschen sind. Das Produkt wird schließlich mit Chloroform/Methanol/25%igem Ammoniak (50/50/5 per Vol.) eluiert. Die vereinigten Eluate werden einrotiert und mit Toluol das restliche Wasser entfernt. Der Rückstand wird in 600 ml Dichlormethan aufgenommen und mit 4 l Aceton versetzt. Die bei -20°C abgeschiedenen Kristalle werden mit kaltem Aceton gewaschen, dann mit Pentan und im Vakuum getrocknet. Die Ausbeute an reinem Hexadecylphosphocholin beträgt 250 g (ca. 70 % bezogen auf Hexadecylglycerin).
Mikroanalyse (MG 407,58):
ber. (%): C 59,27 H 11,37 N 3,29 P 7,28
gef. (%): 58,98 11,31 3,21 7,11

Entsprechende Phosphoethanolamine und Phosphocholine wurden mit Tetradecanol, Octadecanol, Eicosanol, Oleylalkohol, cis-11-Hexadeun-1-ol und Dodecanol hergestellt.

**Beispiel 3**

2-Hexadecylphosphoethanolamin (Phosphorylierung, Ringschluß, Ringöffnung)

Der Ansatz erfolgt wie unter Beispiel 1 beschrieben, jedoch für 0,1 Mol. Um gute Ausbeuten zu erreichen, müssen die Phosphorylierungsbedingungen etwas modifiziert werden, d. h. die Temperatur im Phosphorylierungsschritt wird auf 25°C gesteigert. Sonst wird wie beschrieben verfahren und aufgearbeitet.
Mikroanalyse (MG 365,50)
ber. (%): C 59,15 H 11,03 N 3,83 P 8,48
gef. (%): 58,96 10,91 3,69 8,39

**Beispiel 4**

2-Hexadecylphosphocholin * $1H_2O$ (Methylierung von 3)

Es kann, wie in Vorschrift 2 angegeben vorgegangen, aufgearbeitet und gereinigt werden.
Mikroanalyse (MG 407,58):
ber. (%): C 59,27 H 11,37 N 3,29 P 7,28
gef. (%): 59,14 11,11 3,14 7,09

**Beispiel 5**

Oleylphosphomethylester, Natriumsalz * 1 $H_2O$ (Phosphorylierung, Methanolyse und LiBr-Spaltung)

Der Phosphorylierungsschritt erfolgt wie in Beispiel 1. Zur Methanolyse wird das Reaktionsgemisch mit Methanol (10 Mol; 320 g) und Triethanolamin (1,8 Mol; 180 g) bei 20°C versetzt. Die Methanolyse ist nach 30 Minuten abgeschlossen. Man versetzt mit 1,5l Hexan und 1,5l Wasser, schüttelt gut durch und entfernt das Lösungsmittel von der Hexanphase. Der ölige Rückstand wird mit LiBr (2 Mol; 174 g) in 1,5l Ethylmethylketon unter Rückfluß gekocht. Nach einer Stunde ist die Reaktion vollständig. Man entfernt das Lösungsmittel, nimmt in einem Gemisch aus jeweils 1 l Methanol/Wasser/Chloroform auf, schüttelt gut durch und isoliert die untere Chloroformphase, die das Produkt enthält. Zur Umwandlung in das Natriumsalz behandelt man die Chloroformphase mit 1 l gesättigter NaCl-Lösung. Die Chloroformphase wird isoliert und einrotiert. Man reinigt das Produkt durch Chromatographie an Kieselgel (siehe Beispiel 2).
Mikroanalyse (MG 402,50)
ber. (%): C 56,70 H 10,02 P 7,70
gef. (%): 56,65 9,98 7,45

Entsprechend wurde der Allylester hergestellt. Weiterhin wurden nach dem beschriebenen Verfahren Methyl-und Allylphosphorsäureester von folgenden Alkoholen dargestellt: Tetradecanol, Hexadecanol, Octadecanol und Eicosanol.

**Beispiel 6**

Hexadecylphosphohexylester, Natriumsalz * $1H_2O$ (Phosphorylierung mit Phosphoroxychlorid, Phosphorylierung mit Hexadecylphosphorsäuredichlorid, Methanolyse, Spaltung mit LiBr)

Die Phosphorylierung von Hexadecanol erfolgt wie unter Beispiel 1 beschrieben. Das Reaktionsgcmisch wird direkt unter tropfenweiser Zugabe von Hexanol (1,5 Mol, 303 g) und Triethylamin (1,8 Mol, 180 g) in 1,5 l THF weiter umgesetzt. Die Temperatur wird jetzt auf 30°C erhöht. Nach zwei Stunden ist die Reaktion beendet. Methanolyse erfolgt wie in Beispiel 5 beschrieben, ebenso die LiBr-Spaltung.
Mikroanalyse (MG 446,59)
ber. (%): C 59,17 H 10,83 P 6,94
gef. (%): 59,08 10,74 6,71

Nach diesem Verfahren wurden folgende Alkylester dargestellt: Hexadecylphosphobutyl-,-octyl-, -decyl- und -dodecylester.

**Beispiel 7**

Hexadecylphosphoglykolester, Natriumsalz x $1 H_2O$ (Phosphorylierung, Ringschluß mit Glykol, Ringöffnung)

Die Phosphorylierung erfolgt wie unter Beispiel 1 beschrieben. Das Reaktionsgemisch wird direkt unter tropfenweiser Zugabe von Ethylenglykol (1,5 Mol, 93 g) und Triethanolamin (1,8 Mol, 180 g) in 1,5 l THF weiter umgesetzt. Die Temperatur wird dabei zur Vervollständigung der Ringbildung auf 60°C erhöht. Nach 2 Stunden bei dieser Temperatur ist die Reaktion abgeschlossen. Man filtriert das ausgefallene Triethylaminhydrochlorid über eine Prozellanfritte ab und versetzt das Filtrat unter starkem Rühren bei 20°C mit 1,5 l Wasser. Nach 2 Stunden ist die Hydrolyse beendet. Man entfernt das Lösungsmittel von der oberen THF-Phase durch Einrotieren im Vakuum. Der Rückstand wird mit einem Gemisch Chloroform/Methanol/halbgesättigte NaCl-Lösung versetzt, geschüttelt und Phasenseparation abgewartet. Die untere Chloroformphase enthält das Produkt. Man entfernt das Lösungsmittel und reinigt das Produkt durch Chromatographie (Beispiel 2).
Mikroanalyse (MG 406,48)
ber. (%): C 53,19 H 9,92 P 7,62
gef. (%): 53,07 9,73 7,53

Analog wurden folgende Glykolester hergestellt: Tetradecylphosphoglykolester, Octadecylphosphoglykolester, Oleylphosphoglykolester.

## Beispiel 8

Hexadecylphospho-hydroxyethylamid, Natriumsalz + $1 H_2O$ (Phosphorylierung, Ringschluß mit Ethanolamin, Öffnung mit Kaliumcarbonat in Wasser)

Die Phosphorylierung erfolgt wie unter Beispiel 1 beschrieben, ebenso der Ringschluß. Nach Entfernen des Triethylaminhydrochlorids wird das Filtrat unter starkem Rühren mit 1 l l M Kaliumcarbonatlösung in Wasser versetzt. Nach 1 Stunde ist die Ringöffnung abgeschlossen. Man entfernt in der oberen THF-Phase das Lösungsmittel, nimmt in einem Gemisch aus jeweils 1 l Chloroform/ Methanol/halbgesättigte NaCl-Lösung auf, schüttelt gut durch und trennt die Chloroformphase ab. Nach Entfernen des Lösunosmittels wird das Produkt an Kieselgel chromatographiert und gereinigt.
Mikroanalyse (MG 405,50)
ber. (%): C 53,32 H 10,19 N 3,46 P 7,64
gef. (%): 53,26 10,07 3,21 7,59

Analog wurden folgende Verbindungen hergestellt: Tetradecyl-, Octadecyl-, Oleylphospho-hydroxyethylamid.

## Beispiel 9

Hexadecylphosphoglycerin, Natriumsalz + $H_2O$ (Phosphorylierung mit Phosphoroxychlorid, Phosphorylierung mit dem daraus gebildeten Phosphorsäuredichlorid, Methanolyse, LiBr-Spaltung, Hydrolyse in 70%iger Essigsäure)

Die Phosphorylierung entspricht Beispiel 1. Das Reaktionsgemisch wird direkt unter tropfenweiser Zugabe von 1,2-Isopropyliden-glycerin (1,5 Mol, 198 g) und Triethylamin (1,8 Mol, 180 g) in 1,5 l THF weiter umgesetzt. Die Temperatur wird nach dem Eintropfen auf 30°C erhöht. Die Reaktion ist nach zwei Stunden beendet. Methanolyse erfolgt nach Beispiel 5, ebenso die LiBr-Spaltung. Das Reaktionsprodukt, Natriumsalz, wird in 2 l 70%iger Essigsäure aufgenommen und auf 60°C erwärmt. Das entstehende Aceton wird in leichtem Vakuum (Wasserstrahlvakuum) entfernt. Die Reaktion ist nach 2 Stunden abgeschlossen. Man versetzt mit 2 l Wasser und extrahiert mit 2 l Chloroform. Die Chloroformphase wird mit 2 l 0,5 M Natriumcarbonat-Lösung behandelt und nach Phasenseparation abgetrennt. Man entfernt das Lösungsmittel und chromatographiert an Kieselgel.
Mikroanalyse (MG 436,51)
ber. (%): C 52,28 H 9,70 P 7,10
gef. (%): 52,13 9,59 6,91

Analog wurden folgende Glycerinester hergestellt:
Tetradecyl-, Octadecyl-, Oleylphosphoglycerin.

**Beispiel 10**

Hexadecylphosphorsäure-(N,N)-bis-(chlorethyl)-amid, Na-Salz + $H_2O$ (Phosphorylierung mit Phosphoroxychlorid, Amidbildung mit Bis-(chlorethyl)-amin, Hydrolyse)

Der Phosphorylierungsschritt entspricht Beispiel 1. Das Reaktionsgemisch wird direkt unter tropfenweiser Zugabe von Bis-(chlorethyl)-amin in 1,0 l THF weiter umgesetzt. Danach wird Triethylamin (0,4 Mol, 40 g) in 0,5 l THF zugegeben. Nach 3 Stunden bei 20°C ist die Reaktion beendet. Man trennt das ausgefallene Triethylaminhydrochlorid über einer Porzellanfritte ab und versetzt das Filtrat unter starkem Rühren mit 1 l lM Essigsäure zur Hydrolyse. Nach 4 Stunden wird die obere THF-Phase abgetrennt, vom Lösungsmittel befreit und in jeweils 1 l an Chloroform/Methanol/0,5 M Natriumcarbonat aufgenommen. Die Chloroformphase wird abgenommen, das Lösungsmittel entfernt und das Produkt durch Chromatographie an Kieselgel gereinigt.
Mikroanalyse (MG 486,452)
ber. (%): C 49,38 H 8,91 Cl 14,58 N 2,88 P 6,37
gef. (%): 49,21 8,75 14,11 2,76 6,31

Analog wurden folgende Verbindungen hergestellt:
Tetradecyl-, Octadecyl-, Oleylphosphorsäure-(N,N)-bis-(chlorethyl)-amid.

Beispiele für pharmazeutische Zubereitungen

Beispiel für eine Lösung:

25 g 1-n-Propyloxy-2,3-Propandiol, 12,5 g 1-n-Hexyloxy-2,3-propandiol, 12,5 g 1-n-Nonyloxy-2,3-propandiol, 44 g Wasser und 1 g Phenoxyethanol werden gemischt und 5 g Hexadecylphosphocholin in dieser Mischung gelöst. Die Lösung wird durch Filtration über geeignete Filter von sichtbaren Partikeln befreit.
1 g Lösung enthält 50 mg Hexadecylphosphoclolin.

Beispiel für eine Salbe:

5 g Substanz Hexadecylphosphoglycerin werden in 35 g dickflüssigem Paraffin suspendiert, 30 g emulgierender Cetylstearylalkohol und 30 g weißes Vaselin werden zugesetzt und geschmolzen. Diese Schmelze wird bis zum Erkalten gerührt. Eine homogene Wirkstoffverteilung wird durch Bearbeitung der erkalteten Schmelze mittels eines geeigneten Homogenisiergerätes (zum Beispiel Dreiwalzenstuhl) erreicht.
1 g der hydrophilen Salze enthält 50 mg Hexadecylphosphoglycerin.

Beispiel für eine Emulsion:

11,83 g 1-n-Propyloxy-2,3-propandiol, 5,91 g 1-n-Hexyloxy-2,3-propandiol, 5,91 g 1-n-Nonyloxy-2,3-propandiol, 20,35 g Wasser und 1,0 g Phenoxyethanol werden gemischt und 5 g Hexadecylphosphocholin in dieser Mischung gelöst. Auf einem Wasserbad werden 30 g weißes Vaselin, 15 g Cetylalkohol und 5 g Sorbitanmonopalmitat geschmolzen, auf 70° C erwärmt und die ebenfalls auf 70° C erwärmte Wirkstofflösung mit Hilfe eines hochtourigen Dispergiergerätes in der Fettphase emulgiert. Anschließend wird unter Rühren die Creme auf 30° C abgekühlt.
1 g Wasser in Öl-Creme enthält 50 mg Hexadecylphosphocholin.

Beispiel für Kapseln:

1,25 kg Hexadecylphosphoglycerin werden in 5 kg Chloroform gelöst und in dieser Lösung 1,25 kg Aerosil suspendiert. Anschließend wird das Lösungsmittel im Vakuum abgezogen. Die trockene Masse wird durch ein 1 mm-Sieb gegeben und noch einmal im Vakuum bei 30° C getrocknet, um letzte Lösungsmittelrückstände zu entfernen. Dieses Granulat wird in bekannter Weise auf einer geeigneten Kapselmaschine in Gelatine Hartkapseln der Größe 00 zu 500 mg abgefüllt.
Eine Kapsel enthält 250 mg Hexadecylphosphoglycerin.

Beispiel für ein Lyophilisat:

In 3 Liter Wasser für Injektionszwecke werden unter Stickstoffbegasung 500 g Mannit gelöst, 50 g Hexadecylphosphoglycerin mit Hilfe eines hochtourigen Homogenisiergerätes dispergiert und mit Wasser für Injektionszwecke auf 4 Liter aufgefüllt. Diese milchige Dispersion wird durch Ultraschallbehandlung oder mit Hilfe eines Spalthomogenisators in ein leicht opaleszierendes kolloiddisperses System überführt.
Unter aseptischen Bedingungen wird nun über ein Membranfilter von 0,22 µm Porenweite sterilfiltriert und zu 40 ml in 100 ml-Injektionsflaschen unter Stickstoffbegasung abgefüllt. Die Flaschen versieht man mit Gefriertrocknungsstopfen und lyophilisiert in einer geeigneten Anlage. Nach der Trocknung wird mit sterilem, getrockneten Stickstoff begast und die Flaschen in der Anlage verschlossen. Die Stopfen werden mit einer Bördelkappe gesichert.
Für die intravenöse Anwendung wird das Lyophilisat in 100 ml Wasser für Injektionszwecke rekonstituiert. 1 Flasche enthält 500 mg Hexadecylphosphoglycerin.

**Ansprüche**

1. Arzneimittel,
dadurch gekennzeichnet,
daß es

a) mindestens eine Verbindung der allgemeinen Formel

$R-Y-PO_2^{\ominus}-X-R_1$ I

oder ein physiologisch verträgliches Salz hiervon enthält, wobei in der Formel I
R einen gesättigten oder ungesättigten Kohlenwasserstoffrest mit 12 bis 24 C-Atomen bedeutet, der auch halogensubstituiert sein kann,
X ein Sauerstoffatom, NH oder $NR_2$ und Y ein Sauerstoffatom oder NH ist, $R_1$ eine $C_1$-$C_8$-Alkylgruppe ist, oder worin $R_1$ eine $C_2$-$C_8$-Alkylgruppe darstellt, die ungesättigt und/oder mit Halogen, Amino, $C_1$-$C_6$-Alkylamino, Di-$C_1$-$C_6$-alkylamino, Tri-$C_1$-$C_6$-alkylamino, Hydroxy, Carboxy, $C_3$-$C_8$-Cycloalkyl oder Phenyl substituiert ist, und worin $R_1$ außerdem auch 2-tert.-Butyloxycarbonylaminoethyl, 2-tert.-butyloxycarbonylethyl, 2,3-Isopropylidendioxy-propyl-(1), 2,3-Dibenzyloxy-propyl-(1), 1,3-Dibenzyloxy-propyl-(2) oder N-$C_1$-$C_6$-Alkylamino-$C_2$-$C_6$-alkyl bedeuten kann, wenn X ein Sauerstoffatom ist, und worin $R_1$ außerdem auch 2,3-Dihydroxypropyl-(1) bedeuten kann, wenn X die NH-Gruppe ist,
und $R_2$ eine 2,3-Dihydroxypropyl-(1)-gruppe, eine $C_1$-$C_8$-Alkylgruppe oder eine $C_2$-$C_8$ Alkylgruppe, die ungesättigt und/oder mit Halogen, Amino, $C_1$-$C_6$-Alkylamino, Di-$C_1$-$C_6$-alkylamino, Tri-$C_1$-$C_6$-alkylamino, Hydroxy, Carboxy, $C_3$-$C_8$-Cycloalkyl oder Phenyl substituiert ist, darstellt, und

b) ein Alkylglycerin der allgemeinen Formel II

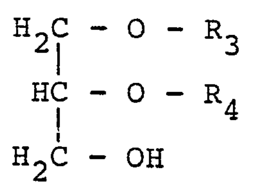

in der einer der Reste $R_3$ und $R_4$ eine Alkylgruppe mit 3 bis 12 C-Atomen und der andere Rest ein H-Atom bedeutet, enthält,
sowie gegebenenfalls weitere übliche pharmazeutische Zusatz-und Verdünnungsmittel.

2. Arzneimittel,
dadurch gekennzeichnet,
daß es mindestens eine Verbindung der allgemeinen Formel

R -Y -$PO_2^{\ominus}$ -X -$R_1$ I'

oder ein physiologisch verträgliches Salz hiervon enthält, wobei in der Formel I'
R einen gesättigten oder ungesättigten Kohlenwasserstoffrest mit 12 bis 24 C-Atomen bedeutet, der auch halogensubstituiert sein kann,
X ein Sauerstoffatom, NH oder $NR_2$ und Y ein Sauerstoffatom oder NH ist,
der Rest $R_1$

a) eine $C_1$-$C_8$-Alkylgruppe, eine ungesättigte $C_3$-$C_8$-Alkylgruppe oder eine gegebenenfalls ungesättigte $C_3$-$C_8$-Alkylgruppe, die durch Halogen, Amino, $C_1$-$C_6$-Alkylamino, Di-$C_1$-$C_6$-alkylamino, Tri-$C_1$-$C_6$-alkylamino, Hydroxy, Carboxy, $C_3$-$C_8$-Cycloalkyl oder Phenyl substituiert ist, darstellt oder

b) eine $C_2$-Alkylgruppe, die durch Halogen, Hydroxy, Carboxy, $C_3$-$C_8$-Cycloalkyl oder Phenyl substituiert ist, darstellt, oder

c) eine ungesättigte $C_2$-Alkylgruppe, die durch Di-$C_1$-$C_6$-alkylamino, Tri-$C_1$-$C_6$ alkylamino, Carboxy, $C_3$-$C_8$-Cycloalkyl oder Phenyl substituiert ist, darstellt, oder

d) eine $C_2$-Alkylgruppe, die durch Amino, $C_1$-$C_6$-Alkylamino, Di-$C_1$-$C_6$-alkylamino oder Tri-$C_1$-$C_6$-alkylamino substituiert ist, darstellt, falls X Sauerstoff, NH oder $NR_2$ und Y die Gruppe NH bedeutet oder falls X die Gruppe NH oder $NR_2$ und Y Sauerstoff bedeutet und R die angegebenen Bedeutungen hat, oder

e) 2-tert.-Butyloxycarbonylaminoethyl, 2-tert.-Butyloxycarbonylethyl, 2,3-Isopropylidendioxy-propyl-(1), 2,3-Dibenzyloxypropyl-(1), 1,3-Dibenzyloxy-propyl-(2) oder N-$C_1$-$C_6$-Alkylamino-$C_2$-$C_6$-alkyl bedeuten kann, wenn X ein Sauerstoffatom ist und Y und R die angegebenen Bedeutungen haben,
oder

f) 2,3-Dihydroxypropyl-(1) bedeuten kann, wenn X die NH-Gruppe ist und Y und R die angegebenen Bedeutungen haben,

und $R_2$ eine 2,3-Dihydroxypropyl-(1)-gruppe, eine $C_1$-$C_8$ Alkylgruppe oder eine $C_2$-$C_8$-Alkylgruppe, die ungesättigt und/oder mit Halogen, Amino, $C_1$-$C_6$-Alkylamino, Di-$C_1$-$C_6$-alkylamino, Tri-$C_1$-$C_6$-alkylamino, Hydroxy, Carboxy, $C_3$-$C_8$-Cycloalkyl oder Phenyl substituiert ist, darstellt,
gegebenenfalls mit zusätzlichen üblichen pharmazeutischen Zusatz-und Verdünnungsmitteln.

3. Arzneimittel nach Anspruch 1 oder 2,
dadurch gekennzeichnet,
daß R ein Alkyl-oder Alkenylrest mit 14 bis 20 C-Atomen, X ein Sauerstoffatom und $R_1$ = Trialkylammoniumethyl mit 1 bis 3 C-Atomen je Alkylgruppe ist.

4. Mittel nach einem oder mehreren der vorangegangenen Ansprüche,
dadurch gekennzeichnet,
daß es zur topischen Behandlung von Hauttumoren 5 bis 200 mg Verbindung gemäß Formel I oder I' pro ml Alkylglycerin enthält.

5. Mittel nach einem oder mehreren der vorangegangenen Ansprüche,
dadurch gekennzeichnet,
daß es ein Alkylglycerin-Gemisch enthält aus Nonyl-beziehungsweise Octylglycerin, Hexylbeziehungsweise Pentylglycerin und Propylbeziehungsweise Ethylglycerin sowie Wasser.

6. Mittel nach einem oder mehreren der vorangegangenen Ansprüche,
dadurch gekennzeichnet,
daß es zur oralen Behandlung von Tumoren als Trinklösung formuliert wird mit einer Tagesdosis zwischen 5 und 100 mg/kg Körpergewicht.

7. Mittel nach einem oder mehreren der vorangegangenen Ansprüche,
dadurch gekennzeichnet,
daß es zur intravenösen Behandlung von Tumoren, eine Verbindung gemäß allgemeiner Formel I oder I' in einer Menge von 5 bis 100 mg/kg Körpergewicht in physiologischer Kochsalzlösung enthält.

8. Verbindungen der allgemeinen Formel

R -Y -$PO_2^{\ominus}$ -X -$R_1$ I'

in der
R einen gesättigten oder ungesättigten Kohlenwasserstoffrest mit 12 bis 24 C-Atomen bedeutet, der auch halogensubstituiert sein kann,
X ein Sauerstoffatom, NH oder $NR_2$ und Y ein Sauerstoffatom oder NH ist,
der Rest $R_1$

a) eine $C_1$-$C_8$-Alkylgruppe, eine ungesättigte $C_3$-$C_8$-Alkylgruppe oder eine gegebenenfalls ungesättigte $C_3$-$C_8$-Alkylgruppe, die durch Halogen, Amino, $C_1$-$C_6$-Alkylamino, Di-$C_1$-$C_6$-alkylamino, Tri-$C_1$-$C_6$-alkylamino, Hydroxy, Carboxy, $C_3$-$C_8$-Cycloalkyl oder Phenyl substituiert ist, darstellt oder

b) eine $C_2$-Alkylgruppe, die durch Halogen, Hydroxy, Carboxy, $C_3$-$C_8$-Cycloalkyl oder Phenyl substituiert ist, darstellt, oder

c) eine ungesättigte $C_2$-Alkylgruppe, die durch Di-$C_1$-$C_6$-alkylamino, Tri-$C_1$-$C_6$-alkylamino, Carboxy, $C_3$-$C_8$-Cycloalkyl oder Phenyl substituiert ist, darstellt, oder

d) eine $C_2$-Alkylgruppe, die durch Amino, $C_1$-$C_6$-Alkylamino, Di-$C_1$-$C_6$-alkylamino oder Tri-$C_1$-$C_6$-alkylamino substituiert ist, darstellt, falls X Sauerstoff, NH oder $NR_2$ und Y die Gruppe NH bedeutet oder falls X die Gruppe NH oder $NR_2$ und Y Sauerstoff bedeutet und R die angegebenen Bedeutungen hat, oder

e) 2-tert.-Butyloxycarbonylaminoethyl, 2-tert.-Butyloxycarbonylethyl, 2,3-Isopropylidendioxy-propyl-(1), 2,3-Dibenzyloxy-propyl-(1), 1,3-Dibenzyloxy-propyl-(2) oder N-$C_1$-$C_6$-Alkylamino-$C_2$-$C_6$-alkyl bedeuten kann, wenn X ein Sauerstoffatom ist und Y und R die angegebenen Bedeutungen haben,
oder

f) 2,3-Dihydroxypropyl-(1) bedeuten kann, wenn X die NH-Gruppe ist und Y und R die angegebenen Bedeutungen haben,

und $R_2$ eine 2,3-Dihydroxypropyl-(1)-gruppe, eine $C_1$-$C_8$-Alkylgruppe oder eine $C_2$-$C_8$-Alkylgruppe, die ungesättigt und/oder mit Halogen, Amino, $C_1$-$C_6$-Alkylamino, Di-$C_1$-$C_6$-alkylamino, Tri-$C_1$-$C_6$-alkyl amino, Hydroxy, Carboxy, $C_3$-$C_8$-Cycloalkyl oder Phenyl substituiert ist, darstellt,
und deren physiologisch verträgliche Salze,
wobei solche Verbindungen ausgenommen sind,
wo in der Formel I' X und Y beide Sauerstoff sind, $R_1$ ein gesättigter oder ungesättigter $C_1$-$C_8$-Alkylrest ist, der auch durch Hydroxy, Amino, $C_1$-$C_6$-Alkylamino, Di-$C_1$-$C_6$-alkylamino oder Tri-$C_1$-$C_6$-alkylamino substituiert sein kann, und R einen gesättigten oder ungesättigten $C_{12}$-$C_{24}$-Alkylrest darstellt.

9. Verfahren zur Herstellung von Verbindungen der Formel

$R-Y-PO_2^{\ominus}-X-R_1$   I

in der

R einen gesättigten oder ungesättigten Kohlenwasserstoffrest mit 12 bis 24 C-Atomen bedeutet, der auch halogensubstituiert sein kann, X ein Sauerstoffatom, NH oder $NR_2$ und Y ein Sauerstoffatom oder NH ist, $R_1$ eine $C_1$-$C_8$-Alkylgruppe ist oder worin $R_1$ eine $C_2$-$C_8$-Alkylgruppe darstellt, die ungesättigt und/oder mit Halogen, Amino, $C_1$-$C_6$-Alkylamino, Di-$C_1$-$C_6$-alkylamino, Tri-$C_1$-$C_6$-alkylamino, Hydroxy, Carboxy, $C_3$-$C_8$-Cycloalkyl oder Phenyl substituiert ist,
und worin $R_1$ außerdem auch 2-tert.-Butyloxycarbonylaminoethyl, 2-tert.-butyloxycarbonylethyl, 2,3-Isopropylidendioxy-propyl-(1), 2,3-Dibenzyloxy-propyl-(1), 1,3-Dibenzyloxy-propyl-(2) oder N-$C_1$-$C_6$-Alkylamino-$C_2$-$C_6$-alkyl bedeuten kann, wenn X ein Sauerstoffatom ist, und worin $R_1$ außerdem auch 2,3-Dihydroxypropyl-(1) bedeuten kann, wenn X die NH-Gruppe ist,
und $R_2$ eine 2,3-Dihydroxypropyl-(1)-gruppe, eine $C_1$-$C_8$-Alkylgruppe oder eine $C_2$-$C_8$-Alkylgruppe, die ungesättigt und/oder mit Halogen, Amino, $C_1$-$C_6$-Alkylamino, Di-$C_1$-$C_6$-alkylamino, Tri-$C_1$-$C_6$-alkylamino, Hydroxy, Carboxy, $C_3$-$C_8$-Cycloalkyl oder Phenyl substituiert ist, darstellt,
und deren physiologisch verträglichen Salzen,
dadurch gekennzeichnet,
daß man eine Verbindung der Formel

$R-Y-\overset{O}{\underset{OZ}{P}}-XR_1$   III

worin R, $R_1$, Y und X die angegebenen Bedeutungen haben, Z Benzyl, $C_1$-$C_6$-Alkyl oder $C_2$-$C_6$-Alkenyl ist, vorhandene Hydroxygruppen, Carboxygruppen, Aminogruppen oder $C_1$-$C_6$-Alkylaminogruppen auch eine übliche Schutzgruppe enthalten können, beziehungsweise 2 benachbarte Hydroxygruppen auch durch ein aliphatisches $C_3$-$C_6$-Keton acetalisiert sein können, in einem inerten Mittel mit Alkalibromiden, Alkalijodiden, niederen Alkylmagnesiumhalogeniden oder Aminen behandelt, gegebenenfalls in den erhaltenen Verbindungen vorhandene Schutzgruppen abspaltet, gegebenenfalls erhaltene Reaktionsprodukte, bei denen $R_1$ ein Halogenatom enthält, mit Ammoniak oder einem Amin der Formel $NR_5R_6R_7$ umsetzt, wobei die Reste $R_5$, $R_6$ und $R_7$ gleich oder verschieden sind und Wasserstoff oder $C_1$-$C_6$-Alkyl bedeuten, und/oder in erhaltenen Produkten mit einer Aminogruppe diese Aminogruppe durch $C_1$-$C_6$-Alkylgruppen alkyliert,
und gegebenenfalls die erhaltenen Produkte in die Salze überführt.

10. Verfahren zur Herstellung von Verbindungen der Formel I'

$R-Y-PO_2^{\ominus}-X-R_1$   I'

in der

R einen gesättigten oder ungesättigten Kohlenwasserstoffrest mit 12 bis 24 C-Atomen bedeutet, der auch halogensubstituiert sein kann, X ein Sauerstoffatom, NH oder $NR_2$ und Y ein Sauerstoffatom oder NH ist, der Rest $R_1$

a) eine $C_1$-$C_8$-Alkylgruppe, eine ungesättigte $C_3$-$C_8$-Alkylgruppe oder eine gegebenenfalls ungesättigte $C_3$-$C_8$-Alkylgruppe, die durch Halogen, Amino, $C_1$-$C_6$-Alkylamino, Di-$C_1$-$C_6$-alkylamino, Tri-$C_1$-$C_6$-alkylamino, Hydroxy, Carboxy, $C_3$-$C_8$-Cycloalkyl oder Phenyl substituiert ist, darstellt oder

b) eine $C_2$-Alkylgruppe, die durch Halogen, Hydroxy, Carboxy, $C_3$-$C_8$-Cycloalkyl oder Phenyl substituiert ist, darstellt, oder

c) eine ungesättigte $C_2$-Alkylgruppe, die durch Di-$C_1$-$C_6$-alkylamino, Tri-$C_1$-$C_6$-alkylamino, Carboxy, $C_3$-$C_8$-Cycloalkyl oder Phenyl substituiert ist, darstellt, oder

d) eine $C_2$-Alkylgruppe, die durch Amino, $C_1$-$C_6$-Alkylamino, Di-$C_1$-$C_6$-alkylamino oder Tri-$C_1$-$C_6$-alkylamino substituiert ist, darstellt, falls X Sauerstoff, NH oder $NR_2$ und Y die Gruppe NH bedeutet oder falls X die Gruppe NH oder $NR_2$ und Y Sauerstoff bedeutet und R die angegebenen Bedeutungen hat, oder

e) 2-tert.-Butyloxycarbonylaminoethyl, 2-tert.-Butyloxycarbonylethyl, 2,3-Isopropylidendioxy-propyl-(1), 2,3-Dibenzyloxy-propyl-(1), 1,3-Dibenzyloxy-propyl-(2) oder N-$C_1$-$C_6$-Alkylamino-$C_2$-$C_6$-alkyl bedeuten kann, wenn X ein Sauerstoffatom ist und Y und R die angegebenen Bedeutungen haben, oder

f) 2,3-Dihydroxypropyl-(1) bedeuten kann, wenn X die NH-Gruppe ist und Y und R die angegebenen Bedeutungen haben,

und $R_2$ eine 2,3-Dihydroxypropyl-(1)-gruppe, eine $C_1$-$C_8$-Alkylgruppe oder eine $C_2$-$C_8$-Alkylgruppe, die ungesättigt und/oder mit Halogen, Amino, $C_1$-$C_6$-Alkylamino, Di-$C_1$-$C_6$-alkylamino, Tri-$C_1$-$C_6$-alkylamino, Hydroxy, Carboxy, $C_3$-$C_8$-Cycloalkyl oder Phenyl substituiert ist, darstellt,
und deren physiologisch verträgliche Salze, wobei solche Verbindungen ausgenommen sind, wo in der Formel I' X und Y beide Sauerstoff sind, $R_1$ ein gesättigter oder ungesättigter $C_1$-$C_8$-Alkylrest ist, der auch durch Hydroxy, Amino, $C_1$-$C_6$-Alkylamino, Di-$C_1$-$C_6$-alkylamino oder Tri-$C_1$-$C_6$-alkylamino substituiert sein kann, und R einen gesättigten oder ungesättigten $C_{12}$-$C_{24}$-Alkylrest darstellt, nach dem vorangegangenen Verfahrensanspruch.

11. Verfahren zur Herstellung von Verbindungen der Formel I' nach einem oder mehreren der vorangegangenen Verfahrensansprüche,
dadurch gekennzeichnet,
daß R ein ungesättigter Kohlenwasserstoffrest mit 12 bis 14 C-Atomen ist, der auch
sein kann, und Y, X, $R_1$ und $R_2$ die angegebenen Bedeutungen haben, und wobei R auch ein gesättigter, gegebenenfalls halogensubstituierter Kohlenwasserstoffrest mit 12 bis 14 C-Atomen sein kann, wenn X die Gruppe NH oder $NR_2$ bedeutet und Y Sauerstoff oder NH ist.

12. Verfahren zur Herstellung eines (antitumorwirksamen) synergistisch wirkenden Arzneimittels,
dadurch gekennzeichnet,
daß man 1 Gewichtsteil von mindestens einer Verbindung der Formel I oder I', worin R, $R_1$, $R_2$, X und Y die angegebenen Bedeutungen haben und die Verbindungen I und I' auch in Form ihrer physiologisch verträglichen Salze vorliegen können, mit 1 bis 30 Gewichtsteilen von mindestens einem Glycerinether der Formel I', worin $R_3$ und $R_4$ die angegebene Bedeutung haben, oder einem Gemisch solcher Glycerinether sowie gegebenenfalls 1 bis 30 Gewichtsteilen Wasser und gegebenenfalls weiteren üblichen Träger- und/oder Verdünnungs-beziehungsweise Hilfsstoffen bei Temperaturen zwischen 20 und 120° C vermischt beziehungsweise homogenisiert und gegebenenfalls die so erhaltene Mischung zur Herstellung von Zubereitungen, die in der Dosierungseinheit 5 bis 2000 mg Wirkstoff der Formel I beziehungsweise I' enthalten, in Hohlzellen entsprechender Größe ausgießt oder in Kapseln entsprechender Größe abfüllt oder granuliert und dann gegebenenfalls unter Zusatz von weiteren üblichen Hilfsstoffen zu Tabletten verpreßt.

13. Verfahren zur Herstellung eines (antitumorwirksamen) synergistisch wirkenden Arzneimittels,
dadurch gekennzeichnet,
daß man 1 Gewichtsteil von mindestens einer Verbindung der Formel I oder I', worin R, $R_1$, $R_2$, X und Y die angegebenen Bedeutungen haben und die Verbindungen I und I' auch in Form ihrer physiologisch verträglichen Salze vorliegen können, mit 1 bis 30 Gewichtsteilen von mindestens einem Glycerinether der Formel II, worin $R_3$ und $R_4$ die angegebene Bedeutung haben, oder einem Gemisch solcher Glycerinether sowie gegebenenfalls 1 bis 30 Ge wichtsteilen Wasser sowie einem oder mehreren der folgenden Stoffe: Stärke, Cellulose, Lactose, Formalin-Casein, modifizierte Stärke, Magnesiumstearat, Calciumhydrogenphosphat, hochdisperse Kieselsäure, Talkum, Phenoxyethanol vermischt, die erhaltene Mischung, gegebenenfalls mit einer wässrigen Lösung, die als Bestandteil mindestens Gelatine, Stärke, Polyvinylpyrrolidon, Vinylpyrrolidon-Vinylacetat-Copolymerisat und/oder Polyoxyethylensorbitanmonooleat enthält, granuliert, das

Granulat gegebenenfalls mit einem oder mehreren der obengenannten Hilfsstoffe homogenisiert, und diese Mischung zu Tabletten verpresst oder in Kapseln abfüllt, wobei die Tabletten oder Kapseln in der Dosierungseinheit jeweils 5 bis 2000 mg Wirkstoff der Formel I enthalten.

14. Verfahren zur Herstellung eines (antitumorwirksamen) synergistisch wirkenden Arzneimittels, dadurch gekennzeichnet,
daß man 1 Gewichtsteil von mindestens einer Verbindung der Formel I oder I', worin R, $R_1$, $R_2$, X und Y die angegebenen Bedeutungen haben und die Verbindungen I und I' auch in Form ihrer physiologisch verträglichen Salze vorliegen können, mit 1 bis 30 Gewichtsteilen von mindestens einem Glycerinether der Formel II, worin $R_3$ und $R_4$ die angegebene Bedeutung haben, oder einem Gemisch solcher Glycerinether sowie gegebenenfalls 1 bis 30 Gewichtsteilen Wasser nach Zusatz von Sojalecithin sowie gegebenenfalls 0,1 -0,5 Gewichtsteilen Phenoxyethanol (bezogen auf einen Gewichtsteil Verbindung I oder I') bei Temperaturen zwischen 33 -37° C in geschmolzenem Hartfett suspendiert und homogenisiert und anschließend die Mischung in Hohlzellen ausgießt, wobei die Dosierungseinheit 5 bis 2000 mg Wirkstoff der Formel I enthält.

15. Verfahren zur Herstellung eines (antitumorwirksamen) synergistisch wirkenden Arzneimittels (Salze, Creme, Emulsion),
dadurch gekennzeichnet,
daß man 1 Gewichtsteil von mindestens einer Verbindung der Formel I oder I', worin R, $R_1$, $R_2$, X und Y die angegebenen Bedeutungen haben und die Verbindungen I und I' auch in Form ihrer physiologisch verträglichen Salze vorliegen können, mit 1 bis 30 Gewichtsteilen von mindestens einem Glycerinether der Formel II, worin $R_3$ und $R_4$ die angegegebene Bedeutung haben, oder einem Gemisch solcher Glycerinether sowie gegebenenfalls 1 bis 30 Gewichtsteilen Wasser bei einer Temperatur zwischen 50 bis 120° C, gegebenenfalls in Gegenwart eines oder mehrerer Emulgatoren und/oder 0,1 -0,5 Gewichtsteilen Phenoxyethanol (bezogen auf 1 Gewichtsteil Verbindung I oder I') mit mindestens einem der folgenden Stoffe homogenisiert: Paraffin, Vaseline, aliphatischer Alkohol mit 12 bis 25 C-Atomen, Sorbitanmonopalmitat, aliphatische Monocarbonsäure mit 15 bis 20 C-Atomen, Polyoxyethylenpolyolfettsäureester, und gegebenenfalls unter Zusatz eines mehrwertigen niederen aliphatischen Alkohols emulgiert.

16. Verfahren zur Herstellung einer (antitumorwirksamen) synergistisch wirkenden Lösung,
dadurch gekennzeichnet,
daß man 1 Gewichtsteil von mindestens einer Verbindung der Formel I oder I', worin R, $R_1$, $R_2$, X und Y die angegebenen Bedeutungen haben und die Verbindungen I und I' auch in Form ihrer physiologisch verträglichen Salze vorliegen können, mit 1 bis 30 Gewichtsteilen von mindestens einem Glycerinether der Formel II, worin $R_3$ und $R_4$ die angegebene Bedeutung haben, oder einem Gemisch solcher Glycerinether sowie gegebenenfalls 1 bis 30 Gewichtsteilen Wasser,gegebenenfalls in Gegenwart von 0,1 -0,5 Gewichtsteilen Phenoxyethanol (bezogen auf einen Gewichtsteil Verbindung I oder I') sowie gegebenenfalls in Anwesenheit eines Emulgators, bei Temperaturen zwischen 30 -100° C auflöst, und gegebenenfalls die so erhaltene Lösung mit soviel Wasser oder Pflanzenöl auffüllt, daß die Endlösung 0,1 -5 Gewichtsprozent an Wirkstoff der Formel I beziehungsweise I' enthält.